(19) Europäisches Patentamt
European Patent Office
Office européen des brevets

(11) EP 3 146 080 B1

(12) EUROPEAN PATENT SPECIFICATION

(45) Date of publication and mention
of the grant of the patent:
06.01.2021 Bulletin 2021/01

(51) Int Cl.:
C12Q 1/6858 (2018.01)

(21) Application number: 15796383.6

(22) Date of filing: 19.05.2015

(86) International application number:
PCT/US2015/031476

(87) International publication number:
WO 2015/179339 (26.11.2015 Gazette 2015/47)

(54) **ALLELE-SPECIFIC AMPLIFICATION USING A COMPOSITION OF OVERLAPPING NON-ALLELE-SPECIFIC PRIMER AND ALLELE-SPECIFIC BLOCKER OLIGONUCLEOTIDES**

ALLELSPEZIFISCHE AMPLIFIZIERUNG MIT EINER ZUSAMMENSETZUNG AUS ÜBERLAPPENDEN NICHT-ALLELSPEZIFISCHEM PRIMER UND ALLELSPEZIFISCHE BLOCKEROLIGONUKLEOTIDE

AMPLIFICATION SPÉCIFIQUE D'UN ALLÈLE AU MOYEN D'UNE COMPOSITION D'AMORCE NON SPÉCIFIQUE D'ALLÈLE DE CHEVAUCHEMENT ET D'OLIGONUCLÉOTIDES BLOQUEURS SPÉCIFIQUES D'UN ALLÈLE

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 19.05.2014 US 201462000114 P

(43) Date of publication of application:
29.03.2017 Bulletin 2017/13

(73) Proprietor: William Marsh Rice University
Houston, TX 77005 (US)

(72) Inventors:
• ZHANG, David Yu
Houston, TX 77030 (US)
• WU, Ruojia
Houston, TX 77054 (US)
• WANG, Juexiao
Houston, TX 77054 (US)

(74) Representative: Potter Clarkson
The Belgrave Centre
Talbot Street
Nottingham NG1 5GG (GB)

(56) References cited:
WO-A1-2013/097173    WO-A1-2014/177540
WO-A2-2011/146403    WO-A2-2012/151560
US-A1- 2009 053 720    US-A1- 2010 009 355

US-A1- 2013 149 695    US-A1- 2014 017 685

• MURDOCK D G ET AL: "The age-related accumulation of a mitochondrial DNA control region mutation in muscle, but not brain, detected by a sensitive PNA-directed PCR clamping based method", NUCLEIC ACIDS RESEARCH, INFORMATION RETRIEVAL LTD, vol. 28, no. 21, 1 November 2000 (2000-11-01), pages 4350-4355, XP002310905, ISSN: 0305-1048
• VESTHEIM ET AL.: 'Blocking primers to enhance PCR amplification of rare sequences in mixed samples - a case study on prey DNA in Antarctic krill stomachs.' FRONT ZOOL vol. 5, no. 12, 20 July 2008, ISSN 1742-9994 pages 1 - 11, XP021038220
• WU LUCIA R ET AL: "Multiplexed enrichment of rare DNA variants via sequence-selective and temperature-robust amplification", NATURE BIOMEDICAL ENGINEERING, NATURE PUBLISHING GROUP UK, LONDON, vol. 1, no. 9, 4 September 2017 (2017-09-04), pages 714-723, XP036428896, DOI: 10.1038/S41551-017-0126-5 [retrieved on 2017-09-04]
• SANTALUCIA J ET AL: "THE THERMODYNAMICS OF DNA TRUCTURAL MOTIFS", ANNUAL REVIEW OF BIOPHYSICS AND BIOMOLECULAR STRUCTURE, ANNUAL REVIEWS, vol. 33, 1 January 2004 (2004-01-01), pages 415-440, XP009053922, ISSN: 1056-8700, DOI: 10.1146/ANNUREV.BIOPHYS.32.110601.141800

Remarks:
    The file contains technical information submitted after the application was filed and not included in this specification

Remarks:
    The file contains technical information submitted after the application was filed and not included in this specification

**Description**

**CROSS-REFERENCE TO RELATED APPLICATIONS**

The present application claims priority to U.S. Provisional Application No. 62/000,114 filed on May 19, 2014.

**SEQUENCE LISTING**

The sequence listing is filed with the application in electronic format only. The sequence listing text file "14-21013-WO(260947.00251)_SL.txt" was created on May 18, 2015, and is 13,144 bytes in size.

**BACKGROUND**

Small differences in DNA and RNA sequence can lead to big differences in overall physical health and wellness of organisms including human beings. For example, a single-base change in a bacterial genome can lead to antibiotic resistance, and a single-base change in a human genome can lead to cancer progression. With the maturation of the genomics field and the accompanying discovery of many nucleic acid biomarker sequences and molecules, there is a strong demand from the biotechnology industry to develop reliable, robust, inexpensive, and precise nucleic acid assays that can discriminate single-base changes. In particular, many PCR-based assays have been developed that are allele-specific.

PCR-based approaches to the selective detection of rare mutations can be broadly classified into two families of approaches: (A) allele-specific primers, and (B) non-allele-specific primers with allele-specific blockers.

Examples of allele-specific primers include the amplification refractory mutation system (ARMS), allele-specific blocker PCR (ASB-PCR), and competitive allele specific TaqMan PCR (castPCR). Examples of (B) include PNA blocker PCR and co-amplification at lower denaturation temperature PCR (COLD-PCR).

Prior to the present invention, allele-specific primers have generally been superior (higher mutation sensitivity) at detecting known single base mutations, while non-allele-specific primers with allele-specific blockers are generally applied for the detection of multiple closely clustered mutations (hotspots) or unknown mutation sequences.

Allele-specific PCR primers such as ARMS, ASB-PCR, castPCR generally possess an allele-specific nucleotide at the 3'-most position of the primer. The allele-specific PCR primers employ the discrimination of the DNA polymerase enzyme to specifically extend properly paired bases, but are limited by the fact that, when an incorrect DNA polymerase extension event does occur, the rare allele nucleotide that was a part of the primer becomes incorporated in the template, and subsequently amplification cycles become non-specific. The allele-specific PCR primers are only specific up to the first incorrect amplification event. Allele-specific detection with non-allele-specific primers requires precise denaturation temperature control, and restricted analysis of smaller sequences. The allele-specific detection with non-allele-specific primers has low mutation sensitivity and is more vulnerable to polymerase-introduced errors.

US2014017685 relates to methods, compositions, and kits for determining the presence/absence of a variant nucleic acid sequence.

Thus, a composition of non-allele-specific primer and allele-specific blocker oligonucleotides with increased allele-specific amplification and improved mutation sensitivity is needed. Therefore, the development of new approaches suitable for the detection of a small amount of target in a large excess of variant is a prerequisite for the diagnostic applications. Thus, the present disclosure provides a composition of non-allele-specific primer and allele-specific blocker oligonucleotides to provide a more accurate and an efficient tool for disease diagnostic applications such as, cancer diagnosis and like.

**SUMMARY**

The present invention is defined in the appended claims. The present disclosure relates to a composition of overlapping a non-allele-specific primer oligonucleotide and an allele-specific blocker oligonucleotide for allele-specific amplification.

The present disclosure provides an oligonucleotide composition including a blocker and a first primer oligonucleotide. The blocker oligonucleotide includes a first sequence having a target-neutral subsequence and a blocker variable subsequence. However, in some instances, the blocker oligonucleotide may not include the blocker variable subsequence if the target nucleic acid to be detected is for the detection of an insertion. The blocker variable subsequence is flanked on its 3' and 5' ends by the target-neutral subsequence and is continuous with the target-neutral subsequence. The first primer oligonucleotide is sufficient to induce enzymatic extension; herein the first primer oligonucleotide includes a second sequence. The second sequence overlaps with the 5' end of the target-neutral subsequence by at least 5 nucleotides such that the second sequence includes an overlapping subsequence and a non-overlapping subsequence.

The second sequence does not include any sequence homologous with the blocker variable subsequence.

The present disclosure further relates to a method for amplification of a target sequence. The method includes obtaining a sample containing one or more copies of a first nucleic acid having a variant sequence and at least one copy of a second nucleic acid; herein the second nucleic acid have the target sequence. The target sequence and variant sequence each includes a homologous subsequence and a variable subsequence. The variable subsequence further includes at least one nucleotide. Furthermore, the variable subsequence of the target sequence is a target-specific subsequence and the variable subsequence of the variant sequence is a non-target specific subsequence.

After obtaining the sample, a blocker oligonucleotide is introduced to the sample. The blocker oligonucleotide includes a first sequence having a target-neutral subsequence and a blocker variable subsequence. The target-neutral subsequence is complementary to a portion of the homologous subsequence and the blocker variable subsequence is complementary to the non-target specific subsequence. Further, the blocker variable subsequence is flanked on its 3' and 5' ends by the target-neutral subsequence and is continuous with the target-neutral subsequence.

Thereafter, a first primer oligonucleotide is introduced to the sample. The first primer oligonucleotide is sufficient to induce enzymatic extension. The first primer oligonucleotide includes a second sequence, which is complementary to a second portion of the homologous subsequence. Further, the second sequence overlaps with the 5' end of the target-neutral subsequence by at least 5 nucleotides such that the second sequence includes an overlapping subsequence and a non-overlapping subsequence. The second sequence does not include any sequence complementary to the variable subsequence.

At the next step, a DNA polymerase, nucleoside triphosphates, and one or more reagents necessary for polymerase-based nucleic acid amplification are introduced to the sample; and the sample is reacted under conditions sufficient to achieve nucleic acid amplification.

An advantage of the present disclosure is improved mutation sensitivity matching and/or exceeding allele-specific primer approaches.

Another advantage of the present disclosure is a simple 2-step thermal cycling protocol with significant (8°C) temperature robustness.

Yet another advantage of the present disclosure is to provide inexpensive DNA primer and blocker reagents without complex backbone or nucleotide modifications.

Yet another advantage of the present disclosure is compatibility with high fidelity enzymes.

Yet another advantage of the present disclosure is that the allele-specific blocker binds more strongly to the variant than to the target, so that the non-allele-specific primer more favourably displace blockers bound to target as compared to blockers bound to variant. The advantage of using non-allele-specific primers is that spuriously amplified variants result in amplification products (amplicons) bearing the variant sequence rather than the target sequence. Thus, specificity is compounded at every cycle.

This summary is provided to introduce disclosure, certain aspects, advantages and novel features of the invention in a simplified form that are further described below in the detailed description. This summary is not intended to identify key features or essential features of the claimed subject matter, nor is it intended to be used to limit the scope of the claimed subject matter.

## BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the disclosure is not limited to specific methods and instrumentalities disclosed herein.

FIG. 1 illustrates a schematic representation of a non-allele-specific primer and an allele-specific blocker for allele-specific amplification in accordance with the present invention.

FIG. 2 illustrates a schematic representation of a thermodynamic design of a primer and a blocker in accordance with the present invention.

FIG. 3 illustrates a schematic representation of calculation of $\Delta G°$ values from sequence in accordance with the present invention. FIG. 3 discloses SEQ ID NOS 1, 33, 1, 34, 2, 33, 2, and 34, respectively, in order of appearance.

FIG. 4 illustrates a schematic representation of rare allele detection in human genomic DNA in accordance with the Example 1. FIG. 4 discloses SEQ ID NOS 3-4, 35, 32, 36, and 32, respectively, in order of appearance.

FIG. 5 illustrates a schematic representation of rare temperature robustness in accordance with the Example 2. FIG. 5 discloses SEQ ID NOS 5-6 and 37-38, respectively, in order of appearance.

FIG. 6A illustrates a schematic representation of increased primer design flexibility in accordance with the Example 3.

FIG. 6B illustrates a schematic representation of the nucleic acid sequences of 4 sets of primer and blocker pairs that amplifies the human SNP rs3789806 in accordance with the Example 3. FIG. 6B discloses SEQ ID NOS 5-12 and 39-40, respectively, in order of appearance.

FIG. 7A illustrates a schematic representation of simulation of effects of $\Delta G^{\circ}_{rxn1}$ with fixed $\Delta\Delta G^{\circ}= 2$ kcal/mol in accordance with the Example 4.

FIG. 7B(I) illustrates a schematic representation of an experimental validation on SMAD7 rs4939827 in accordance with the Example 4. FIG. 7B(I) discloses SEQ ID NOS 3, 13-16, 4, 17, and 35-36, respectively, in order of appearance.

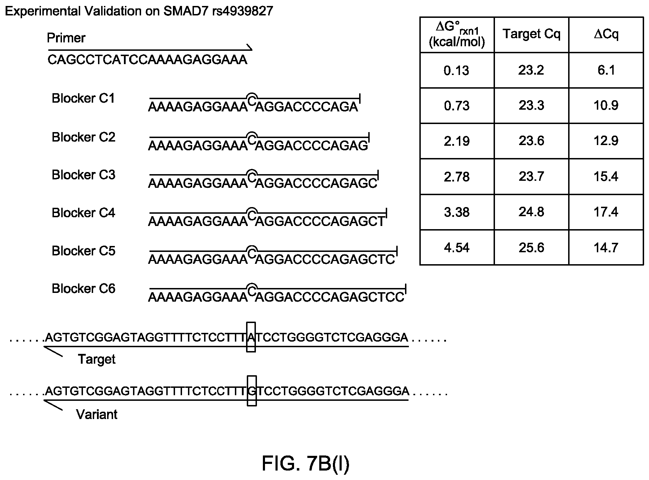

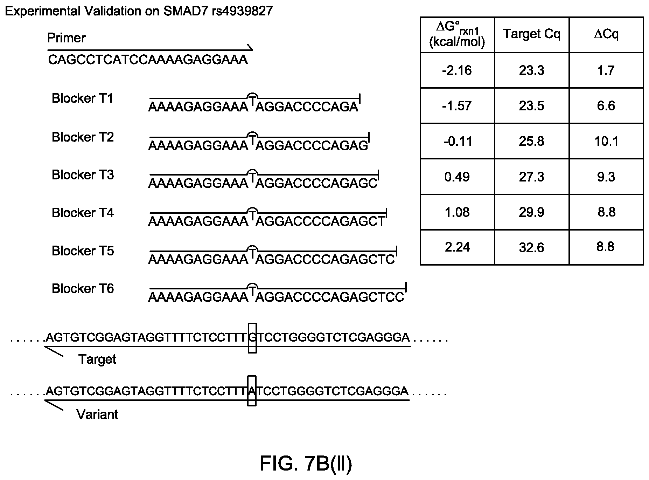

FIG. 7B(II) illustrates a schematic representation of an experimental validation on SMAD7 rs4939827 in accordance with the Example 4. FIG. 7B(II) discloses SEQ ID NOS 3, 18-23, 36, and 35, respectively, in order of appearance.

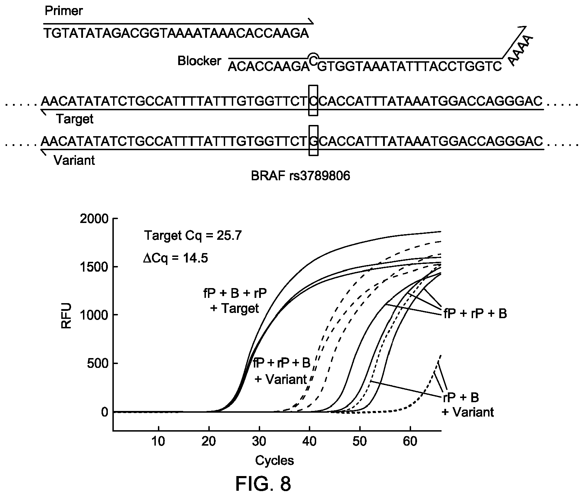

FIG. 8 illustrates a schematic representation of a blocker with 3' non-homologous region in accordance with the Example 5. FIG. 8 discloses SEQ ID NOS 5, 24, and 37-38, respectively, in order of appearance.

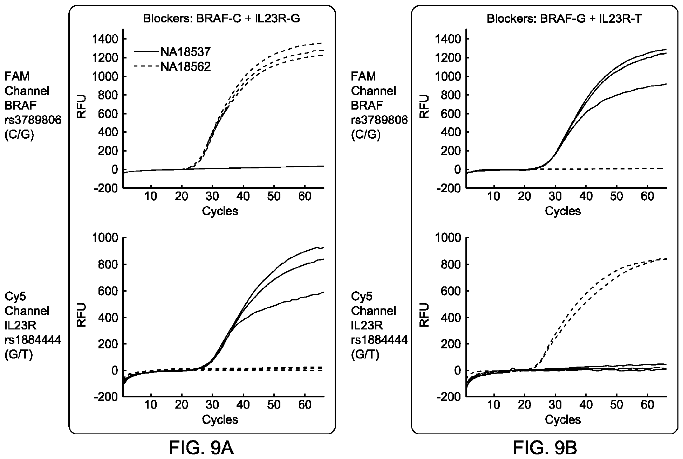

FIG. 9A illustrates a schematic representation of multiplexing using TaqMan® probes in accordance with the Example 6.

FIG. 9B illustrates a schematic representation of multiplexing using TaqMan® probes in accordance with the Example 6.

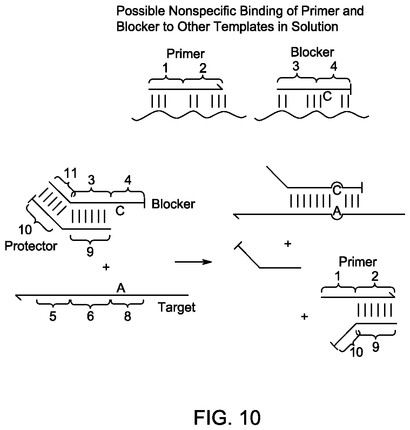

FIG. 10 illustrates a schematic representation of protectors for Primer and Blocker in accordance with the Example 7.

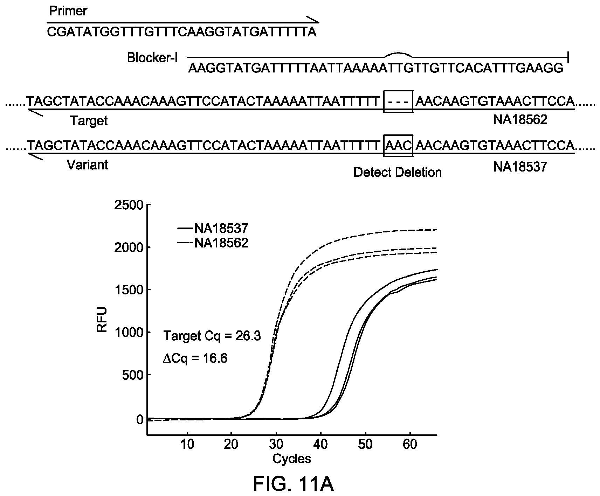

FIG. 11A illustrates a schematic representation of detection of deletion (STAG2 rs200841330) in accordance with the Example 8. FIG. 11A discloses SEQ ID NOS 25-26 and 41-42, respectively, in order of appearance.

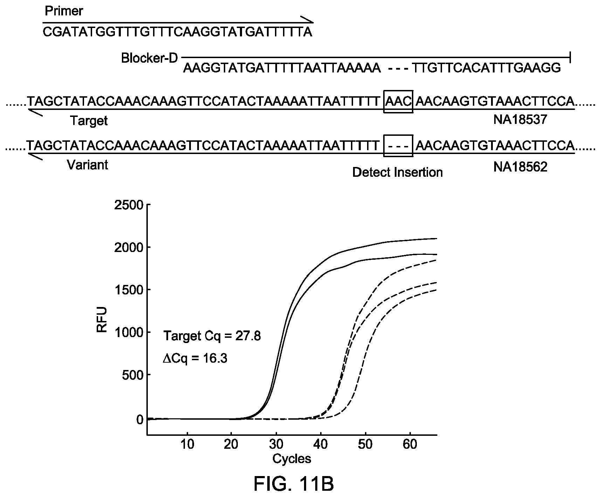

FIG. 11B illustrates a schematic representation of detection of insertion (STAG2 rs200841330) in accordance with the Example 8. FIG. 11B discloses SEQ ID NOS 25, 43, 42, and 41, respectively, in order of appearance.

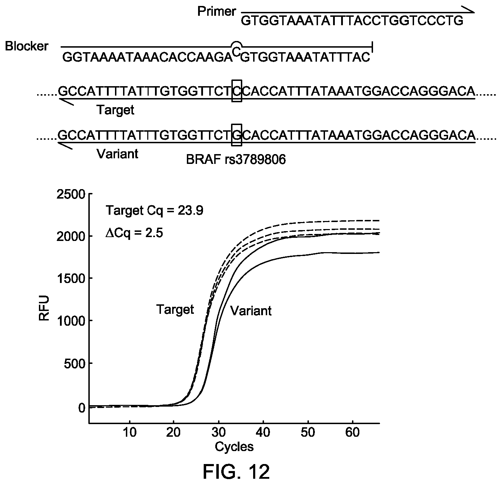

FIG. 12 illustrates a schematic representation of an upstream blocker in accordance with the Example 9. FIG. 12 discloses SEQ ID NOS 27-28 and 44-45, respectively, in order of appearance.

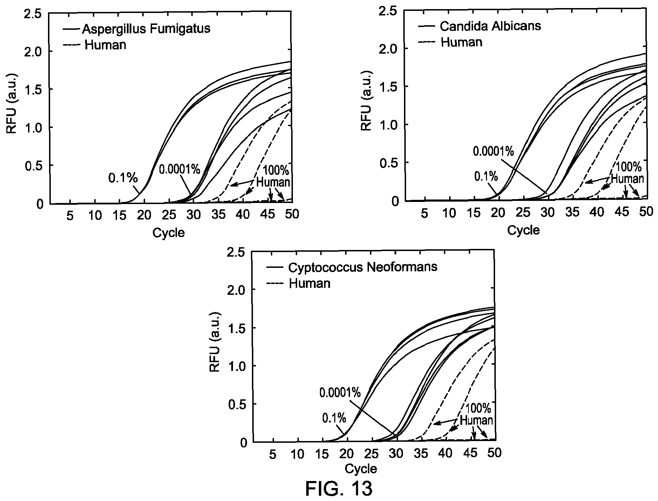

FIG. 13 illustrates a schematic representation of specific amplification of fungal ribosomal RNA-encoding DNA in accordance with the Example 10.

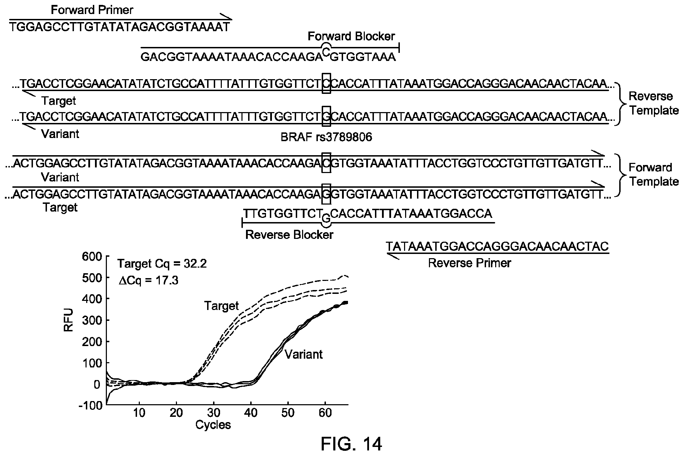

FIG. 14 illustrates a schematic representation of Dual NASP in accordance with the Example 11. FIG. 14 discloses SEQ ID NOS 29, 10, 46-49, and 30-31, respectively, in order of appearance.

## DETAILED DESCRIPTION OF THE INVENTION

The present invention will be described with respect to particular embodiments and with reference to certain drawings, but the invention is not limited thereto, but only by claims. The drawings described are only schematic and are non-limiting. In the drawings, the size of some of the elements may be exaggerated or distorted and not drawn on scale for illustrative purposes. Where the elements of the invention are designated as "a" or "an" in first appearance and designated as "the" or "said" for second or subsequent appearances unless something else is specifically stated.

The present invention now will be described more fully here later to with reference to the accompanying drawings, in which some, but not all embodiments of the invention are shown. Indeed, the invention may be embodied in many different forms and should not be construed as limited to the embodiments set forth herein, rather, these embodiments are provided so that this disclosure satisfies all the legal requirements.

*Definition*

Unless otherwise defined, all technical and scientific terms used herein have the same meaning as commonly understood by one of ordinary skill in the art.

As used herein, the term "blocker oligonucleotide" refers to at least one continuous strand of from about 12 to about 100 nucleotides in length and if so indicated herein, may further include a functional group or nucleotide sequence at its 3' end that prevents enzymatic extension during an amplification process such as polymerase chain reaction.

As used herein, the term "primer oligonucleotide" refers to a molecule comprising at least one continuous strand of from about 12 to about 100 nucleotides in length and sufficient to permit enzymatic extension during an amplification process such as polymerase chain reaction.

As used herein, the term "target-neutral subsequence" refers to a sequence of nucleotides that is complementary to a sequence in both a target nucleic acid and a variant nucleic acid. For example, a desired nucleic acid sequence to be targeted for amplification (target nucleic acid) may exist in a sample with a nucleic acid molecule having a predominantly homologous sequence with the target nucleic acid with the exception of a variable region (variant nucleic acid), such variable region in some instance being only a single nucleotide difference from the target nucleic acid. In this example, the target-neutral subsequence is complementary to at least a portion of the homologous sequence shared between the two nucleic acids, but not the variable region. Thus, as used herein, the term "blocker variable subsequence" refers to a nucleotide sequence of a blocker oligonucleotide which is complementary to the variable region of the variant nucleic.

As used herein, the term "overlapping subsequence" refers to a nucleotide sequence of at least 5 nucleotides of a primer oligonucleotide that is homologous with a portion of the blocker oligonucleotide sequence used in a composition as described herein. The overlapping subsequence of the primer oligonucleotide may be homologous to any portion of the target-neutral subsequence of the blocker oligonucleotide, whether 5' or 3' of the blocker variable subsequence. Thus, the term "non-overlapping subsequence" refers to the sequence of a primer oligonucleotide that is not the overlapping subsequence.

As used herein, the term "target sequence" refers to the nucleotide sequence of a nucleic acid that harbours a desired allele, such as a single nucleotide polymorphism, to be amplified, identified, or otherwise isolated. As used herein, the term "variant sequence" refers to the nucleotide sequence of a nucleic acid that does not harbour the desired allele. For example, in some instances, the variant sequence harbours the wild-type allele whereas the target sequence harbours the mutant allele. Thus, in some instance, the variant sequence and the target sequence are derived from a common locus in a genome such that the sequences of each may be substantially homologous except for a region harbouring the desired allele, nucleotide or group or nucleotides that varies between the two.

The present disclosure relates to an oligonucleotide composition. In an embodiment of the present disclosure, the oligonucleotide composition includes a non-allele-specific primer oligonucleotide and an allele-specific blocker oligonucleotide for allele-specific amplification.

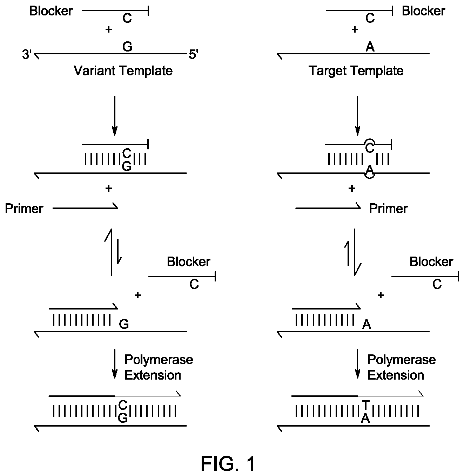

In another embodiment of the present disclosure, the oligonucleotide composition includes a blocker oligonucleotide and a first primer oligonucleotide. The blocker oligonucleotide includes a first sequence having a target-neutral subsequence and a blocker variable subsequence. The blocker variable subsequence is flanked on its 3' and 5' ends by the target-neutral subsequence and is continuous with the target-neutral subsequence. In other words, the blocker variable subsequence may divide the target-neutral subsequence into two portions, a portion to the 3' of the blocker variable subsequence and a portion to the 5'. The first primer oligonucleotide is sufficient to induce enzymatic extension; herein the first primer oligonucleotide includes a second sequence. The second sequence overlaps with the target-neutral subsequence by at least 5 nucleotides such that the second sequence includes an overlapping subsequence and a non-overlapping subsequence. The second sequence does not include the blocker variable subsequence. Thus, the primer oligonucleotide can be characterized as a non-allele specific primer. The overlapping region can be homologous to a portion of the target-neutral subsequence of the blocker oligonucleotide on either the 5' side of the blocker variable subsequence or on the 3' side of the blocker variable subsequence. As shown in FIG. 1, the primer oligonucleotide overlapping subsequence is homologous with the target-neutral subsequence of the blocker on the 5' side of the blocker variable subsequence (denoted as "C" on the blocker). Here, the target-neutral subsequence is the portions of the blocker on either side of the blocker variable subsequence C. FIG. 12 depicts one example where the overlapping subsequence of the primer is homologous with a portion of target-neutral subsequence of the blocker that is 3' of the blocker variable subsequence C.

In yet another embodiment of the present invention, the blocker oligonucleotide further includes a functional group or a non-complementary sequence region at or near the 3' end, which prevents enzymatic extension. In an instance, the functional group of the blocker oligonucleotide is selected from, but is not limited to, the group consisting of a 3-carbon spacer or a dideoxynucleotide.

The second sequence yields a standard free energy of hybridization ($\Delta G°_{PT}$) and the first sequence yields a standard free energy of hybridization ($\Delta G°_{BT}$), which satisfies the following condition:

$$+2 \text{ kcal/mol} \geq \Delta G°_{PT} - \Delta G°_{BT} \geq -8 \text{ kcal/mol}$$

The non-overlapping subsequence yields a standard free energy of hybridization ($\Delta G°_3$), which satisfies the following condition:

$$-4 \text{ kcal/mol} \geq \Delta G°_3 \geq -12 \text{ kcal/mol}$$

In an instance, the second sequence overlaps with the 5' end of the target-neutral subsequence by about 5 nucleotides to about 40 nucleotides. In another instance, the second sequence overlaps with the 5' end of the target-neutral subsequence by about 7 nucleotides to about 30 nucleotides.

In an instance, the concentration of the blocker oligonucleotide is about 2 to about 10,000 times greater than the concentration of the first primer oligonucleotide. In another instance, the concentration of the blocker oligonucleotide is about 5 to about 1,000 times greater than the concentration of the first primer oligonucleotide.

In yet another embodiment of the present disclosure, the oligonucleotide composition further includes a second primer oligonucleotide sufficient to induce enzymatic extension. The second primer oligonucleotide includes a third sequence, which does not overlap the first sequence or second sequence, is target-neutral and is sufficient to use with the first primer oligonucleotide to amplify a region of a nucleic acid using polymerase chain reaction.

In yet another embodiment of the present disclosure, the oligonucleotide composition further includes a second blocker oligonucleotide. The second blocker oligonucleotide includes a fourth sequence having a second target-neutral subsequence and a second blocker variable subsequence. The second blocker variable subsequence has the same sequence as the non-target specific subsequence of the target nucleic acid given that it is typically designed to bind the antisense sequence of the target. The second blocker variable subsequence is flanked on its 3' and 5' ends by the second target-neutral subsequence and is continuous with the second target-neutral subsequence. The third sequence overlaps with the 5' end of the second target-neutral subsequence by at least 5 nucleotides such that the third sequence includes a second overlapping subsequence and a second non-overlapping subsequence.

In yet another embodiment of the present disclosure, the second blocker oligonucleotide further includes a second functional group or a second non-complementary sequence region at or near the 3' end, which prevents enzymatic extension. In an instance, the second functional group of the second blocker oligonucleotide is selected from, but is not limited to, the group consisting of a 3-carbon spacer or a dideoxynucleotide.

The third sequence yields a standard free energy of hybridization ($\Delta G°_{PT2}$) and the fourth sequence yields a standard free energy of hybridization ($\Delta G°_{BT2}$), which satisfies the following condition:

$$+2 \text{ kcal/mol} \geq \Delta G°_{PT2} - \Delta G°_{BT2} \geq -8 \text{ kcal/mol}$$

The second non-overlapping subsequence yields a standard free energy of hybridization ($\Delta G°_6$), which satisfies the following condition:

$$-4 \text{ kcal/mol} \geq \Delta G°_6 \geq -12 \text{ kcal/mol}.$$

In one instance, the third sequence overlaps with the 5' end of the second target-neutral subsequence by about 5 nucleotides to about 40 nucleotides. In another instance, the third sequence overlaps with the 5' end of the second target-neutral subsequence by about 7 nucleotides to about 30 nucleotides.

In any of the above embodiments, the primer oligonucleotide and blocker oligonucleotide may each be from about 12 nucleotides in length to about 100 nucloeotides in length. In any of the above embodiments, the primer oligonucleotide and blocker oligonucleotide may each be from about 15 nucleotides in length to about 90 nucloeotides in length. In any of the above embodiments, the primer oligonucleotide and blocker oligonucleotide may each be from about 20 nucleotides in length to about 80 nucloeotides in length. In any of the above embodiments, the primer oligonucleotide and blocker oligonucleotide may each be from about 20 nucleotides in length to about 70 nucloeotides in length. In any of the above embodiments, the primer oligonucleotide and blocker oligonucleotide may each be from about 20 nucleotides in length to about 60 nucloeotides in length. In any of the above embodiments, the primer oligonucleotide and blocker oligonucleotide may each be 21, 22, 23, 24 , 26, 27, 28, 29, 30, 31, 32, 33, 34, 35, 36, 37, 38, 39, 40, 41, 42, 43, 44, 45, 46, 47, 48, 49, or 50 nucleotides in length.

In an instance, the concentration of the second blocker oligonucleotide is about 2 to about 10,000 times greater than the concentration of the second primer oligonucleotide. In another instance, the concentration of the second blocker oligonucleotide is about 5 to about 1,000 times greater than the concentration of the second primer oligonucleotide.. In another instance, the concentration of the second blocker oligonucleotide is about 10 to about 900 times greater than the concentration of the second primer oligonucleotide. In another instance, the concentration of the second blocker oligonucleotide is about 20 to about 800 times greater than the concentration of the second primer oligonucleotide.. In another instance, the concentration of the second blocker oligonucleotide is about 30 to about 700 times greater than the concentration of the second primer oligonucleotide. In another instance, the concentration of the second blocker oligonucleotide is about 40 to about 600 times greater than the concentration of the second primer oligonucleotide. In another instance, the concentration of the second blocker oligonucleotide is about 50 to about 500 times greater than the concentration of the second primer oligonucleotide. In another instance, the concentration of the second blocker oligonucleotide is about 60 to about 400 times greater than the concentration of the second primer oligonucleotide. In another instance, the concentration of the second blocker oligonucleotide is about 70 to about 300 times greater than the concentration of the second primer oligonucleotide. In another instance, the concentration of the second blocker oligonucleotide is about 80 to about 200 times greater than the concentration of the second primer oligonucleotide. In another instance, the concentration of the second blocker oligonucleotide is about 90 to about 100 times greater than the concentration of the second primer oligonucleotide.

In yet another embodiment of the present disclosure, the oligonucleotide composition further includes a reagent necessary for polymerase chain reaction.

In yet another embodiment of the present disclosure, the oligonucleotide composition further includes a plurality of nucleoside triphosphates.

In yet another embodiment of the present disclosure, the oligonucleotide composition further includes a DNA polymerase or an RNA polymerase.

In yet another embodiment of the present disclosure, the oligonucleotide composition further includes a reagent necessary for polymerase chain reaction, a plurality of nucleoside triphosphates, a DNA polymerase.

In yet another embodiment of the present disclosure, the blocker variable subsequence is a single nucleotide.

The disclosure further relates to a method for amplification of a target sequence. The method includes steps of (a) obtaining a sample containing one or more copies of a first nucleic acid having a variant sequence and at least one copy of a second nucleic acid; herein the second nucleic acid has the target sequence. The target sequence and variant sequence each has a homologous subsequence and a variable subsequence. The variable subsequence further includes at least one nucleotide, but may include 2-5 nucleotides. Furthermore, the variable subsequence of the target sequence is a target-specific subsequence and the variable subsequence of the variant sequence is a non-target specific subsequence.

The method further comprises the step of (b) introducing a blocker oligonucleotide to the sample; herein the blocker oligonucleotide includes a first sequence having a target-neutral subsequence and a blocker variable subsequence. The target-neutral subsequence is complementary to a portion of the homologous subsequence and the blocker variable subsequence is complementary to the non-target specific subsequence. Further, the blocker variable subsequence is flanked on its 3' and 5' ends by the target-neutral subsequence and is continuous with the target-neutral subsequence.

The method further comprises the step of (c) introducing a first primer oligonucleotide to the sample. The first primer oligonucleotide is sufficient to induce enzymatic extension. The first primer oligonucleotide includes a second sequence. Further, the second sequence is complementary to a second portion of the homologous subsequence, and may overlap with the 5' end of the target-neutral subsequence by at least 5 nucleotides such that the second sequence includes an overlapping subsequence and a non-overlapping subsequence. The second sequence does not include any sequence complementary to the variable subsequence.

The method further comprises the step of (d) introducing to the sample a DNA polymerase, nucleoside triphosphates, and one or more reagents necessary for polymerase-based nucleic acid amplification; and then (e) reacting the sample under conditions sufficient to achieve nucleic acid amplification.

In an embodiment of the present disclosure, the blocker oligonucleotide further includes a functional group or a non-complementary sequence region at or near the 3' end, which prevents enzymatic extension. In an instance, the functional group is selected from, but is not limited to, the group consisting of a 3-carbon spacer or a dideoxynucleotide.

The second sequence yields a standard free energy of hybridization ($\Delta G^\circ_{PT}$) and the first sequence yields a standard free energy of hybridization ($\Delta G^\circ_{BT}$), which satisfies the following condition:

$$+2 \text{ kcal/mol} \geq \Delta G^\circ_{PT} - \Delta G^\circ_{BT} \geq -8 \text{ kcal/mol}$$

The non-overlapping subsequence yields a standard free energy of hybridization ($\Delta G^\circ_3$), which satisfies the following condition:

$$-4 \text{ kcal/mol} \geq \Delta G^\circ_3 \geq -12 \text{ kcal/mol}$$

In one instance, the second sequence overlaps with the 5' end of the target-neutral subsequence of the blocker by about 5 nucleotides to about 40 nucleotides (i.e., the "overlapping subsequence). In other words, the second sequence comprises an overlapping subsequence that is homologous with 5 nucleotides to about 40 nucleotides of the target-neutral subsequence of the blocker that is on the 5' side of the blocker variable subsequence of the blocker. In another instance, the second sequence overlaps with the 5' end of the target-neutral subsequence by about 7 nucleotides to about 30 nucleotides. In yet another instance, the second sequence comprises an overlapping subsequence that is homologous to the portion of the target neutral subsequence of the blocker that is on the 3' side of the blocker variable subsequence of the blocker.

In one instance, the concentration of the blocker oligonucleotide introduced into the sample is about 2 to about 10,000 times greater than the concentration of the first primer oligonucleotide introduced into the sample. In another instance, the concentration of the blocker oligonucleotide introduced into the sample is about 5 to about 1,000 times greater than the concentration of the first primer oligonucleotide introduced into the sample. In another instance, the concentration of the blocker oligonucleotide introduced into the sample is about 10 to about 500 times greater than the concentration of the first primer oligonucleotide introduced into the sample. In another instance, the concentration of the blocker oligonucleotide introduced into the sample is about 20 to about 250 times greater than the concentration of the first primer oligonucleotide introduced into the sample. In another instance, the concentration of the blocker oligonucleotide introduced into the sample is about 40 to about 125 times greater than the concentration of the first primer oligonucleotide introduced into the sample. In another instance, the concentration of the blocker oligonucleotide introduced into the sample is about 50 to about 100 times greater than the concentration of the first primer oligonucleotide introduced into the sample. In another instance, the concentration of the blocker oligonucleotide introduced into the sample is about 300 to about 400 times greater than the concentration of the first primer oligonucleotide introduced into the sample. In another instance, the concentration of the blocker oligonucleotide introduced into the sample is about 500 to about 600 times greater than the concentration of the first primer oligonucleotide introduced into the sample.

In another embodiment of the present invention, the DNA polymerase is a thermostable DNA polymerase.

In yet another embodiment of the present disclosure, the method further includes the conditions sufficient to achieve nucleic acid amplification by exposing the sample to at least 10 cycles. Each cycle has at least 2 different temperature exposures, one temperature exposure of at least 85°C, and one temperature exposure of no more than 75°C.

In yet another embodiment of the present disclosure, the method further includes the step of introducing to the sample an enzyme selected from the group consisting of a nicking enzyme, a recombinase, a helicase, a RNAse a reverse transcriptase, or any combination thereof.

In yet another embodiment of the present disclosure, the method further includes a step of introducing a second primer oligonucleotide into the sample. The second primer oligonucleotide includes a third sequence. Further, the third sequence does not overlap with the variable subsequence. Furthermore, the third sequence is target-neutral and is sufficient to use with the first primer oligonucleotide to amplify a region of a nucleic acid having the target sequence.

In yet another embodiment of the present disclosure, the method further includes a step of introducing a second blocker oligonucleotide to the sample. The second blocker oligonucleotide includes a fourth sequence; herein the fourth sequence has a second target-neutral subsequence and a second blocker variable subsequence. Further, the second blocker variable subsequence is the complementary sequence to the blocker variable subsequence. Furthermore, the second blocker variable subsequence is flanked on its 3' and 5' ends by the second target-neutral subsequence and is continuous with the second target-neutral subsequence. In addition, the third sequence overlaps with the 5' end of the second target-neutral subsequence by at least 5 nucleotides such that the third sequence includes a second overlapping subsequence and a second non-overlapping subsequence.

In yet another embodiment of the present disclosure, the second blocker oligonucleotide further includes a second functional group or a second non-complementary sequence region at or near the 3' end, which prevents enzymatic extension. In an instance, the second functional group is selected from, but is not limited to, the group consisting of a 3-carbon spacer or a dideoxynucleotide.

The third sequence yields a standard free energy of hybridization ($\Delta G^{\circ}_{PT2}$) and the fourth sequence yields a standard free energy of hybridization ($\Delta G^{\circ}_{BT2}$), which satisfies the following condition:

$$+2 \text{ kcal/mol} \geq \Delta G^{\circ}_{PT2} - \Delta G^{\circ}_{BT2} \geq -8 \text{ kcal/mol}$$

The second non-overlapping subsequence yields a standard free energy of hybridization ($\Delta G^{\circ}_6$), which satisfies the following condition:

$$-4 \text{ kcal/mol} \geq \Delta G^{\circ}_6 \geq -12 \text{ kcal/mol}$$

In an instance, the third sequence overlaps with the 5' end of the second target-neutral subsequence by about 5 nucleotides to about 40 nucleotides. In another instance, the third sequence overlaps with the 5' end of the second target-neutral subsequence by about 7 nucleotides to about 30 nucleotides.

In an instance, the concentration of the second blocker oligonucleotide introduced into the sample is about 2 to about 10,000 times greater than the concentration of the second primer oligonucleotide introduced into the sample. In another instance, the concentration of the second blocker oligonucleotide introduced into the sample is about 5 to about 1,000 times greater than the concentration of the second primer oligonucleotide introduced into the sample.

In yet another embodiment of the present disclosure, the method for amplification of a target sequence further includes a step of removing an aliquot from the sample; and repeating steps (b) through (e) defined above.

FIG. 1 illustrates a schematic representation of the non-allele-specific primer and the allele-specific blocker for allele-specific amplification. A nucleic acid reagent mixture comprises two oligonucleotide species, a primer (primer oligonucleotide) and a variant specific blocker (blocker oligonucleotide), which act together to enable reliable rare target amplification in conjunction with a polymerase-based amplification method. The blocker may be modified at the 3' end with a non-extensible modification, such as a dideoxynucleotide or a 3-carbon linker. The sequences of the primer and blocker are rationally designed based on the thermodynamics of their hybridization to the target nucleic acid sequence and the variant nucleic acid sequence. In some embodiments, the blocker is present in a significantly higher concentration than the primer, so that the preponderance of the target and the variant nucleic acid sequences bind to blocker before binding to primer. The primer binds transiently to the blocker-target or blocker-variant molecules, and possesses a probability for displacing the blocker in binding to the target or variant. Because the blocker sequence is specific to the variant target, its displacement from the variant is less thermodynamically favourable than its displacement from the target. Thus, the non-allele-specific primer amplifies the target sequence with higher yield/efficiency than it amplifies the variant sequence. The non-allele-specific nature of the primer means that spuriously amplified variant sequence bear the variant allele, rather than the target allele, so that subsequent amplification cycles also exhibit amplification bias in favour of the target.

High concentration of the variant-specific blocker results in higher probability of blocker binding to both the target and the variant first, before the primer has an opportunity to bind. The primer initiates binding, to both the target-blocker or variant-blocker complex, via a unique region not overlapping with the blocker, and subsequently possibly displaces the blocker via a process of enzyme-free strand displacement.

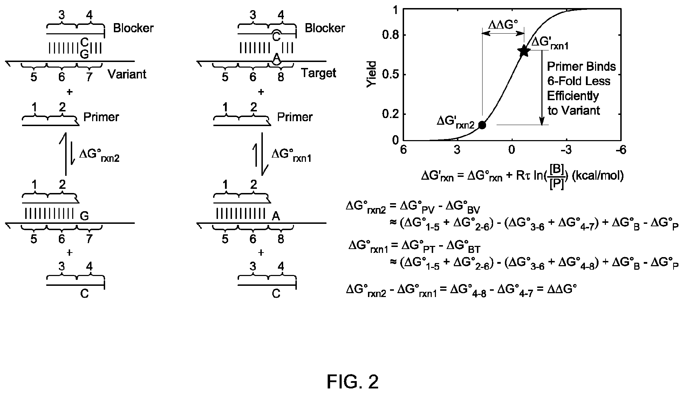

FIG. 2 illustrates a schematic representation of a thermodynamic design of a primer and a blocker. The sequences of the primer and the blocker are rationally designed to achieve desirable reaction thermodynamics. Here, the primer, the blocker, the target, and the variant nucleic acid sequences are subdivided into regions comprising continuous nucleotides, denoted by the numbers 1 through 8 in Fig. 2. The standard free energy of hybridization between the primer and the target ($\Delta G^\circ_{PT}$) and the standard free energy of hybridization between the blocker and the target ($\Delta G^\circ_{BT}$) satisfies:

$$+2 \text{ kcal/mol} \geq \Delta G^\circ_{PT} - \Delta G^\circ_{BT} \geq -8 \text{ kcal/mol}$$

The primer and blocker systems that satisfy the above inequality generate a large difference in the hybridization yields between primer bound to the target and primer bound to the variant in each cycle, resulting in a target-specific amplification.

The process of enzyme-free strand displacement is guided by the relative thermodynamics of the primer binding versus the blocker binding. The blocker hybridizes more favourably to the variant (standard free energy of binding $\Delta G^\circ_{BV}$) than to the target (standard free energy of binding $\Delta G^\circ_{BT}$, $\Delta G^\circ_{BT} > \Delta G^\circ_{BV}$ because more negative $\Delta G^\circ$ indicates stronger favourability). In another embodiment of the present invention, the primer hybridizes equally favourably to the variant (standard free energy of binding $\Delta G^\circ_{PV}$) as to the target (standard free energy of binding $\Delta G^\circ_{PT}$, $\Delta G^\circ_{PV} = \Delta G^\circ_{PT}$).

The reaction of the primer displacing the blocker in binding to the target, BT + P $\Leftrightarrow$ PT + B, has a standard free energy $\Delta G^\circ_{rxn1} = \Delta G^\circ_{PT} - \Delta G^\circ_{BT}$, which is more negative (more favourable) than that of the reaction of the primer displacing the blocker in binding to the variant, BV + P $\Leftrightarrow$ PV + B, which has standard free energy $\Delta G^\circ_{rxn2} = \Delta G^\circ_{PV} - \Delta G^\circ_{BV}$. For good performance of the present invention, the primer and blocker usually should be designed so that $\Delta G^\circ_{rxn1} \leq 0$ and $\Delta G^\circ_{rxn2} \geq 0$. However, because the relative concentrations of the blocker and the primer can influence the equilibrium distribution and effective thermodynamics of the reaction, the $\Delta G^\circ_{rxn1}$ and $\Delta G^\circ_{rxn2}$ guidelines are not absolute.

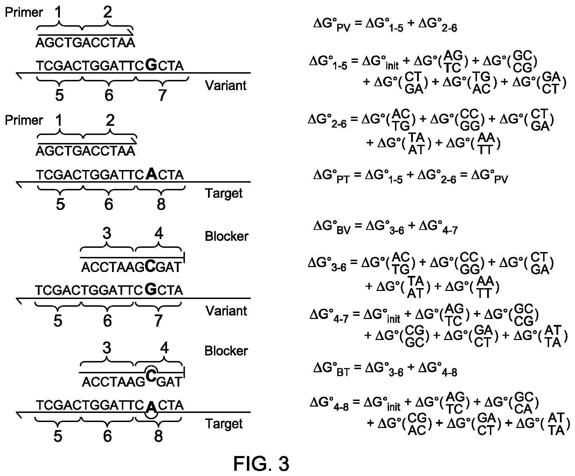

FIG. 3 illustrates a schematic representation of calculation of $\Delta G^\circ$ values from sequence. There exist different conventions for calculating the $\Delta G^\circ$ of different region interactions; shown in FIG. 3 are exemplary energy calculations based on the nearest neighbour model. The $\Delta G^\circ_{1-5}$, $\Delta G^\circ_{4-7}$, and $\Delta G^\circ_{4-8}$ terms further include the standard free energy of hybridization initiation ($\Delta G^\circ_{init}$), and the extra base stack adjoining the sixth region. Standard free energies, $\Delta G^\circ$, calculated using other methods may result in the same $\Delta G^\circ_{PT}$, $\Delta G^\circ_{PV}$, $\Delta G^\circ_{BT}$, and $\Delta G^\circ_{BV}$ values, though the thermodynamics of individual regions (e.g. $\Delta G^\circ_{3-6}$) may differ. The illustrative examples are intended to show the method of $\Delta G^\circ$ calculation used in the present invention, and are not intended to be suggestive of sequences for an assay; the listed sequences may be too short to stably bind. The calculation of $\Delta G^\circ_{PT}$, $\Delta G^\circ_{PV}$, $\Delta G^\circ_{BT}$, and $\Delta G^\circ_{BV}$ from the primer sequence, blocker sequence, target sequence, variant sequence, operational temperature, and operational buffer conditions are known to those skilled in the art.

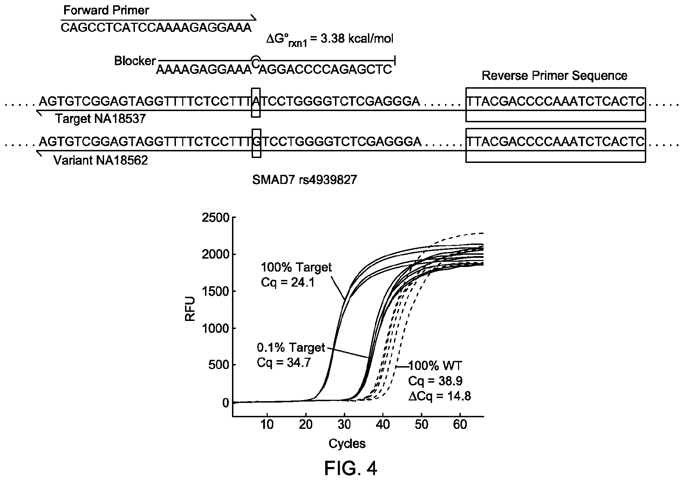

FIG. 4 shows a probe and a blocker design of the present invention and the subsequences around SMAD7 gene locus of two human repository samples NA18537 and NA18562. The design of a primer/blocker set designed to specifically amplify the A variant of the SMAD7 single nucleotide polymorphism (SNP). The blocker is designed to be perfectly complementary to the variant template bearing a G-variant of the SMAD7 SNP, and is functionalized at the 3' end with a 3-carbon linker (C3) that prevents enzymatic extension by the Taq DNA polymerase. A reverse primer binds to the 3' of the forward primer, and is not specific to either allele. For the sake of explanation of terminology used to describe various subsequences of the primar and blocker, the oligonucleotides can be described as follows. The blocker oligonucleotide comprises a target-neutral subsequence which includes the sequence to the 5' and 3' of the cytosine nucleotide that is depicted out of alignement with the remainder of the sequence - the cytosine nucleotide in this instance represents the blocker variable subsequence of the blocker. The overlapping subsequence of the primer oligonucleotide comprises sequence that is homologous to the blocker sequence on the 5' side of the blocker variable subsequence (the cytosine).

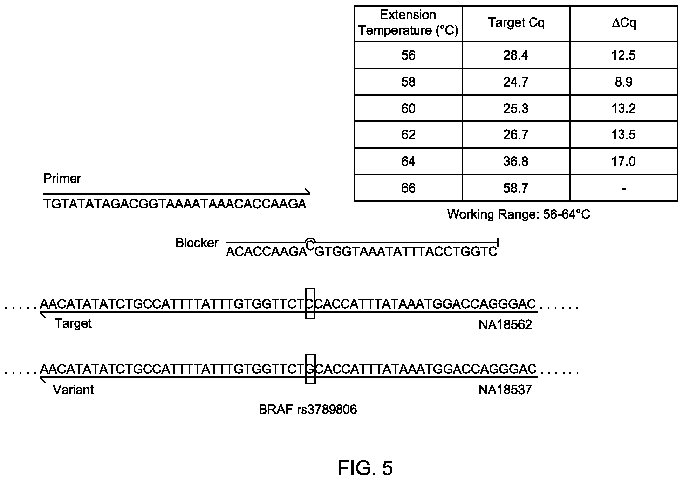

FIG. 5 shows the performance of a primer-blocker pair targeting human SNP rs3789806 (C/G) C allele at annealing/extension temperature ranging from 56°C to 66°C.

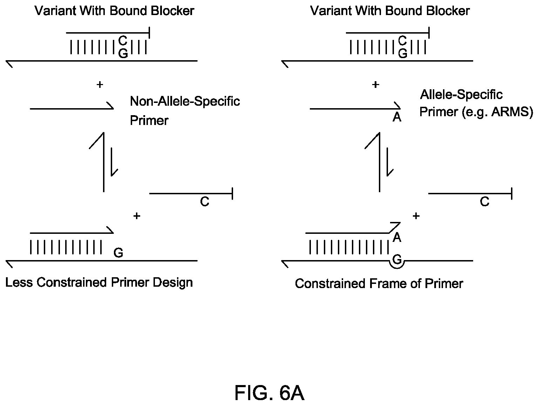

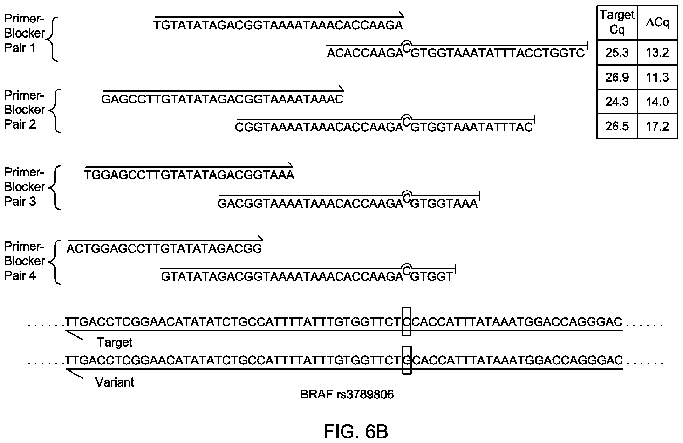

FIG. 6A and FIG. 6B shows primer designs with more design space to avoid undesired interactions.

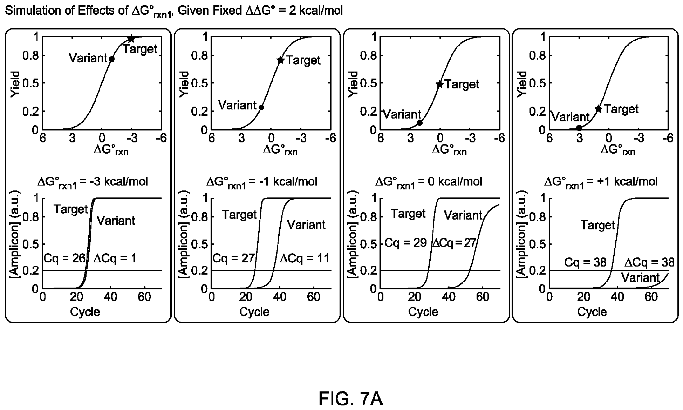

FIG. 7A, and FIG. 7B shows controlling the allele-specific PCR behaviour via design of $\Delta G°_{rxn1}$. Simulations in FIG. 7A suggest that when the primer and blocker are designed so that $\Delta G°_{rxn1}$ is more positive, $\Delta Cq$ values are greater. However, at more positive values of $\Delta G°_{rxn1}$, the amplification of the target is also slowed, resulting in a larger value of Cq. Simulations assume a fixed $\Delta\Delta G°$ of 2 kcal/mol. Top sub graphs show the predicted effects of the different values of $\Delta G°_{rxn1}$ (stars) and $\Delta G°_{rxn2}$ (dots) on the hybridization yields. The bottom subgraphs show simulated amplification curve of the target and the variant for each primer and blocker pair. In all cases depicted, the concentration ratio of blocker to primer is 20:1. FIG. 7B(I) and FIG. 7B(II) show experimental results of $\Delta G°_{rxn1}$ effects on amplification selectivity. Different $\Delta G°_{rxn1}$ values were achieved via lengthening or shortening the 3' end of the blocker. As expected, more positive $\Delta G°_{rxn1}$ results in larger Cq for both the target and the variant. We observed an optimal intermediate $\Delta G°_{rxn1}$ resulting in largest $\Delta Cq$ and relatively small Cq delay (Cq < 28) for the target. Unlike the simulations, $\Delta Cq$ did not increase monotonically with $\Delta G°_{rxn1}$. The formation of primer-dimers and nonspecific amplification produces a Cq ceiling.

The kinetic simulations of the PCR process expressed as:

$$\text{Tfn}+1 = \text{Tfn} + \text{Pn} \cdot [\text{Pn} / (\text{Pn} + \text{Bn}) + Y(\Delta G°_{rxn1}) \cdot \text{Bn} / (\text{Pn} + \text{Bn})] \cdot \text{Trn}$$

$$\text{Trn}+1 = \text{Trn} + \text{Rn} \cdot \text{Tfn}$$

$$\text{Vfn}+1 = \text{Vfn} + \text{Pn} \cdot [\text{Pn} / (\text{Pn} + \text{Bn}) + Y(\Delta G°_{rxn2}) \cdot \text{Bn} / (\text{Pn} + \text{Bn})] \cdot \text{Vrn}$$

$$\text{Vrn}+1 = \text{Vrn} + \text{Rn} \cdot \text{Vfn}$$

where Tfn is the normalized concentration of the forward strand of the target at cycle n, Trn is the normalized concentration of the reverse strand of the target at cycle n, Vfn is the normalized concentration of the forward strand of the variant at cycle n, Vrn is the normalized concentration of the forward strand of the variant at cycle n, Pn is the normalized concentration of the forward primer at cycle n, Bn is the normalized concentration of the blocker at cycle n, Rn is the normalized concentration of the reverse primer at cycle n, and $Y(\Delta G°)$ is the hybridization yield of a displacement reaction given $\Delta G°$ reaction standard free energy. Simulation results are shown in FIG. 7A.

FIG. 8 shows the blocker with a non-homologous sequence at the 3' end rather than a non-extensible chemical functionalization. Because the non-homologous sequences do not bind to the downstream region of either the target or the variant, the non-homologous region effectively prevents enzymatic extension. The target Cq and $\Delta Cq$ values are similar to those shown in Example 1, indicating that like functional groups such as a 3-carbon spacer or a dideoxynucleotide, a non-homologous sequence at the 3' end can effectively prevent enzymatic extension Here fP, rP, and B represent the forward primer, the reverse primer, and the blocker (for the forward primer), respectively.

In standard blocking PCR, only the forward primer is biased to amplify the target sequence; the reverse primer amplifies both the target and the variant roughly equally. Amplification specificity can be further improved (with roughly a quadratic improvement in mutation sensitivity) if both the forward and the reverse primers were biased to amplify only the target sequence. To achieve dual target-specific amplification, the forward and reverse primers are designed to be separated by a short distance, so that they overlap with a variant-specific blocker. In the limit, the primer-binding regions are separated by a single nucleotide whose identity varies between the target and the variant. The forward and reverse variant-specific blockers are partially complementary to each other because they bind to complementary strands of the target, and both span the variation region. The blockers are designed so that, despite their partial complementarity, at the operational conditions, the majority of blocker molecules are not hybridized to each other.

FIG. 9A and FIG. 9B, each shows a primer-blocker systems designed for human BRAF rs3789806 and IL23R rs1884444 SNPs were multiplexed in the same reaction. TaqMan® probes targeting the corresponding downstream of the blocker binding regions were designed and used as the readout mechanism.

FIG. 10 shows nonspecific binding of primer or blocker to other primers, blockers, or templates results in nonspecific amplification. The lower panel provides a design of a protector that suppresses the nonspecific binding of the primer and blocker. Protector oligonucleotides are partially complementary to the primer and blocker sequences. The presence of a stoichiometric excess of protectors result in both the primer and the blocker being partially double-stranded. The blocker possesses a new region 11, whose sequence is non complementary to region 5 on the nucleic acid sequence, and the protector possesses a new region 10, whose sequence is complementary to region 11.

FIG. 11A and FIG. 11B, each shows primer and blocker designs for detecting a deletion and an insertion. The primer is non-target-specific, this method can also be used to detect deletion or insertion with uncertain number of bases or uncertain position.

FIG. 12 shows an inferior implementation of the present invention by placing the primer at the 3' of the SNP position and blocker at the 5' of the primer. This implementation generates hybridization specificity only in the first cycle, so the ΔCq is smaller than the preferred design.

FIG. 13 demonstrates selective amplification of fungal 18S DNA subsequences of 3 pathogenic fungi species in large excess of homologous human DNA. Because fungal 18S subsequences and its homologous human subsequence differ in multiple regions, we designed corresponding blocker species for both forward primer and reverse primer to maximize amplification specificity.

FIG. 14 describes the use of target-specific amplification for both the forward and the reverse primer, by using two sets of variant-allele blockers, one for each primer. The blockers are partially complementary to each other, but at the operational conditions are not predominately hybridized to one another. The blockers and primers are designed so that the forward primer cannot extend off the reverse blocker, and the reverse primer cannot extend off the forward blocker. FIG. 14 shows preliminary experimental results.

The primer and blocker combinations may be applied to other enzymatic amplification assays for nucleic acids, including but not limited to Nicking Enzyme Amplification Reaction (NEAR), Loop-mediated Isothermal Amplification (LAMP), and Rolling Circle Amplification (RCA).

The present invention is particularly suitable for the detection of a small amount of target in a large excess of variant. For cancer diagnostics applications of the present invention, the variant may refer to the wildtype DNA sequence, and the target may refer a cancer DNA sequence. For infectious disease diagnostics applications of the present invention, the variant may refer to the pathogen DNA sequence, and the target may refer to a homologous human DNA sequence.

*Examples*

Example 1

FIG. 4 shows an Example 1 of the present invention for the probe and the blocker design and the subsequences around SMAD7 gene locus of two human repository samples NA18537 and NA18562. Healthy human single nucleotide polymorphisms (SNP) rs4939827 (C/T) in SMAD7 gene locus were chosen for proof-of-concept validations. T allele as target and C allele variant were arbitrarily designated. Human repository genomic DNA samples NA18562 (homozygous of C allele) and NA18537 (homozygous of T allele) were purchased from Coriell®, and the corresponding nucleotide sequences were obtained from 1000 Genomes website. To prevent unintended polymerization, the blocker was modified with a C3 spacer at the 3' end. qPCR experimental results distinguished 0.1% target (99.9% variant) from 0% target (100% variant). The 0.1% target sample was prepared by mixing 0.1% NA18537 with 99.9% NA18562. The average difference of quantification cycle (ΔCq) between 100% variant and 100% target was 14.8, and that between 100% variant (denoted as "WT" in the graph) and 0.1% target was 4.2. All experiments were performed in a 96-well plate in the Bio-Rad CFX96™ qPCR machine. In each well, 200 nM primer, 2 μM blocker, and 20 ng human genomic DNA were mixed in the Bio-rad iTaq™ SYBR® Green Supermix. After a 3 min initiation process at 95°C, 65 cycles of denaturing step at 95°C for 10 seconds and annealing/extension step at 60°C for 30 seconds were performed. Example experiments shown in FIG. 5-12 used a similar protocol.

Table-1

| Gene | SNP | Blocker | Cq numbers | | |
|------|-----|---------|---------|---------|-----|
| | | | NA18537 | NA18562 | ΔCq |
| BRAF | rs3789806 GorC | - | 23.2 | 23.4 | 0.2 |
| | | C | 38.5 | 25.3 | 13.2 |
| | | G | 30.1 | 42.7 | 12.6 |

(continued)

| Gene | SNP | Blocker | Cq numbers | | |
|---|---|---|---|---|---|
| | | | NA18537 | NA18562 | $\Delta$Cq |
| EGF | rs11568849 CorA | - | 22.5 | 22.3 | 0.2 |
| | | A | 34.7 | 26.3 | 8.4 |
| | | C | 24.7 | 39.33 | 14.6 |
| IL23R | rs1884444 GorT | - | 22.2 | 22.4 | 0.2 |
| | | T | 36.1 | 24 | 12.1 |
| | | G | 28.3 | 35.4 | 7.1 |
| ALK | rs2246745 A or T | - | 22.6 | 22.3 | 0.3 |
| | | A | 23.2 | 33 | 9.8 |
| | | T | 33.2 | 24.1 | 9.1 |

Table-1 shows examples of allele specific amplification results for four set of SNP pairs in human genomic DNA samples NA18562 and NA18537, drawn from the 1000 Genomes database. For each SNP pair, a primer and blocker design for each allele variant were designed and tested. The base identities shown in the "Blocker" column indicates the allele that was being suppressed (variant), and "-" means no blocker was added. In all cases, large $\Delta$Cq values between the two alleles, ranging from 7.1 to 14.6 were observed. All the experiments were performed in triplicates using 400 nM of each primer and 4 $\mu$M of blocker in Bio-rad iTaq™ SYBR® Green Supermix. The performance of thermodynamically driven designs without any empirical optimization of primer and blocker sequences or experimental conditions were shown by the results.

Example 2

FIG. 5 shows an Example 2 of the present invention for the performance of a primer-blocker pair targeting human SNP rs3789806 (C/G) C allele at annealing/extension temperature ranging from 56°C to 66°C. The Example 2 showed good allele specific amplification from 56°C to 64°C and indicated at least 8°C of temperature robustness. 400 nM of each primer and 4 $\mu$M blocker were used, and other experimental conditions other than annealing/extension temperature were the same as described in Example 1.

Example 3

FIG. 6A and FIG. 6B show an Example 3 of the present invention. The designs of the Primers shown in the present invention provided more design space to avoid undesired interactions. The nucleic acid sequences of four sets of primer and blocker pairs that each specifically amplifies the human SNP rs3789806 C allele exhibited $\Delta$Cq > 11. 400 nM of each primer and 4 $\mu$M blocker were used, and other experimental conditions were the same as described in Example 1.

Example 4

FIG. 7A, FIG. 7B(I) and FIG. 7B(II) show an Example 4 of the present invention for controlling the allele-specific PCR behaviour via design of $\Delta G^{\circ}_{rxn1}$. Simulations in FIG. 7A suggested that when the primer and blocker were designed so that $\Delta G^{\circ}_{rxn1}$ was more positive, $\Delta$Cq values were greater. However, at more positive values of $\Delta G^{\circ}_{rxn1}$, the amplification of the target was slowed and resulted in a larger value of Cq. Simulations were assumed as fixed $\Delta\Delta G^{\circ}$ of 2 kcal/mol. The top sub graphs predicted the effects of the different values of $\Delta G^{\circ}_{rxn1}$ (stars) and $\Delta G^{\circ}_{rxn2}$ (dots) on the hybridization yields. The bottom sub graphs predicted simulated amplification curve of the target and the variant for each primer and blocker pair. In all cases depicted, the concentration ratio of blocker to primer was 20:1. FIG. 7B(I) and FIG. 7B(II) show experimental results of $\Delta G^{\circ}_{rxn1}$ effects on amplification selectivity. Different $\Delta G^{\circ}_{rxn1}$ values were achieved via lengthening or shortening the 3' end of the blocker. As expected, more positive $\Delta G^{\circ}_{rxn1}$ resulted in larger Cq for both the target and the variant. An optimal intermediate $\Delta G^{\circ}_{rxn1}$ resulted in largest $\Delta$Cq and relatively small Cq delay (Cq < 28) for the target. Unlike the simulations, $\Delta$Cq did not increase monotonically with $\Delta G^{\circ}_{rxn1}$ because of the formation of primer-dimers and nonspecific amplification produced by a Cq ceiling. 200 nM of each primer and 2 $\mu$M blocker were used, and other experimental conditions were same as described in Example 1.

Example 5

FIG. 8 shows an Example 5 of the present invention for the blocker with a non-homologous sequence at the 3' end rather than a non-extensible chemical functionalization. Because the non-homologous sequences do not bind to the downstream region of either the target or the variant, the non-homologous region effectively prevents enzymatic extension. 400 nM of each primer and 4 $\mu$M blocker were used, and other experimental conditions were the same as described in Example 1. The target Cq and $\Delta$Cq values were similar to those shown in FIG. 4, indicating that like functional groups such as a 3-carbon spacer or a didexoynucleotide, a non-homologous sequence at the 3' end effectively prevents enzymatic extension. In FIG. 8, fP, rP, and B represent the forward primer, the reverse primer, and the blocker (for the forward primer), respectively.

Example 6

FIG. 9A and FIG. 9B show an Example 6 of the present invention. For a real-time PCR implementation of our non-allele-specific primer and allele-specific blocker, a reverse primer was also needed, which was not allele-specific. SYBR® Green Supermix and TaqMan® were used in the experiments and the results were not significantly different between the two. The Primer-blocker systems designed for human BRAF rs3789806 and IL23R rs1884444 SNPs were multiplexed in the same reaction. TaqMan® probes targeting the corresponding downstream of the blocker binding regions were designed and used as the readout mechanism. The TaqMan® probe for BRAF rs3789806 amplicons was modified with a FAM fluorophore, an Iowa Black FQ quencher, and an internal ZEN quencher, and the TaqMan® probe for IL23R rs1884444 amplicons was modified with a Cy5 fluorophore and an Iowa Black RQ quencher. FIG. 9A shows the amplification results of multiplexed detection of BRAF rs3789806 G allele and IL23R rs1884444 T allele. The base identities denoted which allele the blockers were suppressing (variant). FIG. 9B shows multiplexed detection of BRAF rs3789806 C allele and IL23R rs1884444 G allele. In each experiment, the genomic DNA sample was mixed with 400 nM of each primer, 4 $\mu$M of each blocker, and 200 nM of each TaqMan® probe in Bio-rad iQ Supermix. The qPCR protocol was the same as indicated in Example 1.

Example 7

FIG. 10 shows an Example 7 of the present invention for nonspecific binding of primer or blocker to other primers, blockers, or templates. The Example 7 resulted in nonspecific amplification. The lower panel provided a design of a protector that suppressed the nonspecific binding of the primer and blocker. The blocker possessed a new region 11, whose sequence was non complementary to region 5 on the nucleic acid sequence, and the protector possessed a new region 10, whose sequence was complementary to region 11.

Example 8

FIG. 11A and FIG. 11B show an Example 8 of the present invention for primer and blocker designs for detecting a deletion and an insertion. Del rs200841330 was used to show proof-of-concept results. 400 nM of each primer and 4 $\mu$M blocker were used, and other experimental conditions were same as indicated in Example 1. Since the primer is non-allele-specific, this method can also be used to detect deletion or insertion with uncertain number of bases or uncertain position.

Example 9

FIG. 12 shows an Example 9 of the present invention by placing the primer to the 3' of the SNP position and blocker to the 5' of the primer. The Example 9 generated hybridization specificity only in the first cycle, so the $\Delta$Cq was smaller than the preferred design. The primer concentration was 400 nM, and the blocker concentration was 4 $\mu$M. Experimental conditions were the same as indicated in Example 1.

Example 10

FIG. 13 shows an Example 10 of the present invention for selective amplification of fungal 18S DNA subsequences of 3 pathogenic fungi species in large excess of homologous human DNA. Because fungal 18S subsequences and its homologous human subsequence differ in multiple regions, corresponding blocker species for both forward primer and reverse primer to maximize amplification specificity were designed. gBlock fragments (sequence verified 400bp double-stranded DNA, IDT) of 18S subsequences from 3 fungi species and the consensus sequence in human were used in the experiments. 60,000 copies (0.1%), 60 copies (0.0001%) and 0 copies (100% Human) of each fungal DNA gBlock fragment were separately mixed with 60,000,000 copies of human DNA. In all cases, results detected rare sequences down to 1 part in 1 million. 200 nM of each primer and 1 μM of each blocker were used, and other experimental conditions were the same as indicated in Example 1.

Example 11

FIG. 14 shows an Example 11 of the present invention. FIG. 14 describes the use of allele-specific amplification for both the forward and the reverse primer; two sets of variant-allele blockers, one for each primer were used. The blockers were partially complementary to each other, but at the operational conditions did not support predominant hybridization to one another. The blockers and primers were designed so that the forward primer cannot extend off the reverse blocker, and the reverse primer cannot extend off the forward blocker. FIG. 14 displayed preliminary experimental results.

The foregoing description of specific embodiments of the present disclosure has been presented for purpose of illustration and description. The exemplary embodiment was chosen and described in order to best explain the principles of the invention and its practical application, to thereby enable others skilled in the art to best utilize the invention and various embodiments with various modifications are suited to the particular use contemplated.

**Claims**

1. A method for amplification of a target sequence comprising the steps of:

   a. providing a sample containing one or more copies of a first nucleic acid comprising a variant sequence and possibly containing at least one copy of a second nucleic acid comprising the target sequence, wherein the target sequence and variant sequence each comprise a homologous subsequence and a variable subsequence, wherein the variable subsequence comprises at least one nucleotide, and wherein the variable subsequence of the target sequence is a target-specific subsequence and the variable subsequence of the variant sequence is a non-target specific subsequence;

   b. designing a DNA blocker oligonucleotide, wherein the DNA blocker oligonucleotide comprises a first sequence comprising a target-neutral subsequence and a blocker variable subsequence, wherein the target-neutral subsequence is complementary to a portion of the homologous subsequence and the blocker variable subsequence is complementary to the non-target specific subsequence, wherein the blocker variable subsequence is flanked on its 3' and 5' ends by the target-neutral subsequence and is continuous with the target-neutral subsequence, and wherein the DNA blocker oligonucleotide comprises a non-complementary sequence region at the 3' end, which prevents enzymatic extension; and

   c. designing a first DNA primer oligonucleotide wherein the first DNA primer oligonucleotide comprises a second sequence, wherein the second sequence is complementary to a second portion of the homologous subsequence, wherein the second sequence overlaps the target-neutral subsequence of the blocker oligonucleotide by at least 5 nucleotides such that the second sequence comprises an overlapping subsequence and a non-overlapping subsequence, and wherein the second sequence does not include any sequence complementary to the variable subsequence; wherein the second sequence of the first DNA primer oligonucleotide is designed to yield a standard free energy of hybridization ($\Delta G°_{PT}$) to the target sequence, and the first sequence of the DNA blocker oligonucleotide is designed to yield a standard free energy of hybridization ($\Delta G°_{BT}$) to the target sequence, which satisfies the following condition: +2 kcal/mol $\geq \Delta G°_{PT} - \Delta G°_{BT} \geq$ -8 kcal/mol; and wherein the non-overlapping subsequence is designed to yield a standard free energy of hybridization ($\Delta G°_{3}$) to the target sequence, which satisfies the following condition: -4 kcal/mol $\geq \Delta G°_{3} \geq$ -12 kcal/mol; and

d. providing the DNA blocker oligonucleotide and the first DNA primer oligonucleotide as designed in steps (b) and (c); and

e. introducing the DNA blocker oligonucleotide and the first DNA primer oligonucleotide to the sample, wherein the first DNA primer oligonucleotide is sufficient to induce enzymatic extension; and

f. introducing to the sample a DNA polymerase, nucleoside triphosphates, and one or more reagents necessary for polymerase-based nucleic acid amplification; and

g. reacting the sample under conditions sufficient to achieve nucleic acid amplification.

2. The method of claim 1 wherein the DNA polymerase is a thermostable DNA polymerase, and wherein the conditions sufficient to achieve nucleic acid amplification comprise exposing the sample to at least 10 cycles, wherein each cycle comprises at least 2 different temperature exposures, one temperature exposure of at least 85 °C, and one temperature exposure of no more than 75 °C.

3. The method of claim 1 further comprising the step of introducing to the sample an enzyme selected from the group consisting of a nicking enzyme, a recombinase, a helicase, a RNAse, a reverse transcriptase, or any combination thereof.

4. The method of claim 1 wherein the overlapping subsequence comprises a portion of the 5' end of the target-neutral subsequence, wherein said portion is from about 5 nucleotides to about 40 nucleotides, or optionally, is from about 7 nucleotides to about 30 nucleotides.

5. The method of claim 1 wherein the concentration of the DNA blocker oligonucleotide introduced into the sample is about 2 to about 10,000 times greater than the concentration of the first DNA primer oligonucleotide introduced into the sample, optionally, wherein the concentration of the DNA blocker oligonucleotide is about 5 to about 1000 times greater than the concentration of the first DNA primer oligonucleotide, optionally wherein the concentration of the DNA blocker oligonucleotide is about 2 to about 5 times greater than the concentration of the first DNA primer oligonucleotide.

**Patentansprüche**

1. Verfahren für eine Amplifikation einer Zielsequenz, das die folgenden Schritte umfasst:

a. Bereitstellen einer Probe, die eine oder mehrere Kopien einer ersten Nukleinsäure enthält, die eine Variantensequenz umfasst, und möglicherweise mindestens eine Kopie einer zweiten Nukleinsäure enthält, die die Zielsequenz umfasst, wobei die Zielsequenz und die Variantensequenz jeweils eine homologe Teilsequenz und eine variable Teilsequenz umfassen, wobei die variable Teilsequenz mindestens ein Nukleotid umfasst, und wobei die variable Teilsequenz der Zielsequenz eine zielspezifische Teilsequenz ist und die variable Teilsequenz der Variantensequenz eine nicht zielspezifische Teilsequenz ist;

b. Entwerfen eines DNA-Blocker-Oligonukleotids, wobei das DNA-Blocker-Oligonukleotid eine erste Sequenz umfasst, die eine zielneutrale Teilsequenz und eine Blocker-variable Teilsequenz umfasst, wobei die zielneutrale Teilsequenz zu einem Abschnitt der homologen Teilsequenz komplementär ist und die Blocker-variable Teilsequenz zu der nicht zielspezifischen Teilsequenz komplementär ist, wobei die Blocker-variable Teilsequenz an ihren 3'- und 5'-Enden durch die zielneutrale Teilsequenz flankiert wird und mit der zielneutralen Teilsequenz kontinuierlich ist, und wobei das DNA-Blocker-Oligonukleotid eine nicht komplementäre Sequenzregion am 3'-Ende umfasst, die eine enzymatische Verlängerung verhindert; und

c. Entwerfen eines ersten DNA-Primer-Oligonukleotids, wobei das erste DNA-Primer-Oligonukleotid eine zweite Sequenz umfasst, wobei die zweite Sequenz zu einem zweiten Abschnitt der homologen Teilsequenz komplementär ist, wobei die zweite Sequenz die zielneutrale Teilsequenz des Blocker-Oligonukleotids um mindestens 5 Nukleotide derart überlappt, dass die zweite Sequenz eine überlappende Teilsequenz und eine nicht überlappende Teilsequenz umfasst, und wobei die zweite Sequenz keine zu der variablen Teilsequenz komplementäre Sequenz einschließt; wobei die zweite Sequenz des ersten DNA-Primer-Oligonukleotids entworfen ist, um

eine freie Standardhybridisierungsenergie ($\Delta G^\circ_{PT}$) an die Zielsequenz zu liefern, und die erste Sequenz des DNA-Blocker-Oligonukleotids entworfen ist, um eine freie Standardhybridisierungsenergie ($\Delta G^\circ_{BT}$) an die Zielsequenz zu liefern, die die folgende Bedingung erfüllt: +2 kcal/mol $\geq \Delta G^\circ_{PT}$ - $\Delta G^\circ_{BT} \geq$ -8 kcal/mol; und wobei die nicht überlappende Teilsequenz entworfen ist, um eine freie Standardhybridisierungsenergie ($\Delta G^\circ_3$) an die Zielsequenz zu liefern, die die folgende Bedingung erfüllt: -4 kcal/mol $\geq \Delta G^\circ_3 \geq$ -12 kcal/mol; und

d. Bereitstellen des DNA-Blocker-Oligonukleotids und des ersten DNA-Primer-Oligonukleotids, wie in den Schritten (b) und (c) entworfen; und

e. Einführen des DNA-Blocker-Oligonukleotids und des ersten DNA-Primer-Oligonukleotids in die Probe, wobei das erste DNA-Primer-Oligonukleotid ausreicht, um eine enzymatische Verlängerung herbeizuführen; und

f. Einführen einer DNA-Polymerase, Nukleosidtriphosphate und eines oder mehrerer Reagenzien, die für eine Nukleinsäureamplifikation auf Polymerasebasis erforderlich sind, in die Probe; und

g. Umsetzen der Probe unter Bedingungen, die ausreichen, um die Nukleinsäureamplifikation zu erreichen.

2. Verfahren nach Anspruch 1, wobei die DNA-Polymerase eine thermostabile DNA-Polymerase ist und wobei die Bedingungen, die ausreichen, um die Nukleinsäureamplifikation zu erreichen, ein Aussetzen der Probe gegenüber mindestens 10 Zyklen umfassen, wobei jeder Zyklus mindestens 2 verschiedene Temperaturaussetzungen umfasst, eine Temperaturaussetzung von mindestens 85 °C und eine Temperaturaussetzung von nicht mehr als 75 °C.

3. Verfahren nach Anspruch 1, das ferner den Schritt des Einführens eines Enzyms in die Probe umfasst, das aus der Gruppe ausgewählt ist, die aus einem Nicking-Enzym, einer Rekombinase, einer Helikase, einer RNAse, einer reversen Transkriptase oder einer beliebigen Kombination davon besteht.

4. Verfahren nach Anspruch 1, wobei die überlappende Teilsequenz einen Abschnitt des 5'-Endes der zielneutralen Teilsequenz umfasst, wobei der Abschnitt etwa 5 Nukleotide bis etwa 40 Nukleotide oder optional etwa 7 Nukleotide bis etwa 30 Nukleotide beinhaltet.

5. Verfahren nach Anspruch 1, wobei die Konzentration des in die Probe eingeführten DNA-Blocker-Oligonukleotids etwa 2- bis etwa 10.000-mal höher als die Konzentration des ersten in die Probe eingeführten DNA-Primer-Oligonukleotids ist, optional wobei die Konzentration des DNA-Blocker-Oligonukleotids etwa 5- bis etwa 1000-mal höher als die Konzentration des ersten DNA-Primer-Oligonukleotids ist, optional wobei die Konzentration des DNA-Blocker-Oligonukleotids etwa 2- bis etwa 5-mal höher als die Konzentration des ersten DNA-Primer-Oligonukleotids ist.

**Revendications**

1. Procédé d'amplification d'une séquence cible comprenant les étapes comprenant :

a. la fourniture d'un échantillon contenant une ou plusieurs copies d'un premier acide nucléique comprenant une séquence variante et contenant éventuellement au moins une copie d'un second acide nucléique comprenant la séquence cible, la séquence cible et la séquence variante comprenant chacune une sous-séquence homologue et une sous-séquence variable, la sous-séquence variable comprenant au moins un nucléotide, et la sous-séquence variable de la séquence cible étant une sous-séquence spécifique cible et la sous-séquence variable de la séquence variante étant une sous-séquence spécifique sans cible spécifique ;

b. la conception d'un oligonucléotide bloqueur d'ADN, l'oligonucléotide bloqueur d'ADN comprenant une première séquence comprenant une sous-séquence neutre cible et une sous-séquence variable bloquante, la sous-séquence neutre cible étant complémentaire d'une partie de la sous-séquence homologue et la sous-séquence variable bloquante étant complémentaire de la sous-séquence sans cible spécifique, la sous-séquence variable bloquante étant flanquée à ses extrémités 3' et 5' par la sous-séquence neutre cible et étant continue avec la sous-séquence neutre cible, et l'oligonucléotide bloqueur d'ADN comprenant une région de séquence non complémentaire à l'extrémité 3', qui empêche l'extension enzymatique ; et

c. la conception d'un premier oligonucléotide d'amorce d'ADN, le premier oligonucléotide d'amorce d'ADN comprenant une seconde séquence, la seconde séquence étant complémentaire d'une seconde partie de la sous-séquence homologue, la seconde séquence chevauchant la sous-séquence neutre cible de l'oligonucléotide bloqueur par au moins 5 nucléotides de sorte que la seconde séquence comprend une sous-séquence chevauchante et une sous-séquence non chevauchante, et la seconde séquence ne comportant aucune séquence complémentaire de la sous-séquence variable ; la seconde séquence du premier oligonucléotide d'amorce d'ADN étant conçue pour produire une énergie libre standard d'hybridation ($\Delta G^\circ_{PT}$) à la séquence cible, et la première séquence de l'oligonucléotide bloqueur d'ADN étant conçue pour produire une énergie libre standard

d'hybridation ($\Delta G^\circ_{BT}$) à la séquence cible, qui satisfait la condition suivante : +2 kcal/mol $\geq \Delta G^\circ_{PT}$- $\Delta G^\circ_{BT}\geq$ -8 kcal/mol ; et la sous-séquence non chevauchante étant conçue pour produire une énergie libre standard d'hybridation ($\Delta G^\circ_3$) à la séquence cible, qui satisfait la condition suivante : -4 kcal/mol $\geq \Delta G^\circ_3\geq$ -12 kcal/mol ; et

d. la fourniture de l'oligonucléotide bloqueur d'ADN et du premier oligonucléotide d'amorce d'ADN tel que conçu dans les étapes (b) et (c) ; et

e. l'introduction de l'oligonucléotide bloqueur d'ADN et du premier oligonucléotide d'amorce d'ADN dans l'échantillon, le premier oligonucléotide d'amorce d'ADN étant suffisant pour induire une extension enzymatique ; et

f. l'introduction dans l'échantillon d'une ADN polymérase, de nucléosides triphosphates et d'un ou plusieurs réactifs nécessaires à l'amplification des acides nucléiques à base de polymérase ; et

g. la réaction de l'échantillon dans des conditions suffisantes pour obtenir une amplification de l'acide nucléique.

2. Procédé selon la revendication 1, dans lequel l'ADN polymérase est un ADN polymérase thermostable, et dans lequel les conditions suffisantes pour obtenir une amplification d'acide nucléique comprennent l'exposition de l'échantillon à au moins 10 cycles, chaque cycle comprenant au moins 2 expositions à des températures différentes, une exposition à une température d'au moins 85 °C, et une exposition à une température ne dépassant pas 75 °C.

3. Procédé selon la revendication 1, comprenant en outre l'étape l'introduction dans l'échantillon d'une enzyme choisie dans le groupe constitué par une enzyme de coupure, une recombinase, une hélicase, une ARNase, une transcriptase inverse ou toute combinaison de celles-ci.

4. Procédé selon la revendication 1, dans lequel la sous-séquence chevauchante comprend une partie de l'extrémité 5' de la sous-séquence neutre cible, ladite partie étant d'environ 5 nucléotides à environ 40 nucléotides, ou éventuellement, étant d'environ 7 nucléotides à environ 30 nucléotides.

5. Procédé selon la revendication 1, dans lequel la concentration de l'oligonucléotide bloqueur d'ADN introduit dans l'échantillon est d'environ 2 à environ 10 000 fois supérieure à la concentration du premier oligonucléotide d'amorce d'ADN introduit dans l'échantillon, éventuellement, la concentration de l'oligonucléotide bloqueur d'ADN étant environ 5 à environ 1 000 fois supérieure à la concentration du premier oligonucléotide d'amorce d'ADN, éventuellement la concentration de l'oligonucléotide bloqueur d'ADN étant environ 2 à environ 5 fois supérieure à la concentration du premier oligonucléotide d'amorce d'ADN.

FIG. 1

$$\Delta G'_{rxn} = \Delta G^\circ_{rxn} + R\tau \ln\left(\frac{[B]}{[P]}\right) \text{ (kcal/mol)}$$

$$\Delta G^\circ_{rxn2} = \Delta G^\circ_{PV} - \Delta G^\circ_{BV}$$
$$\approx (\Delta G^\circ_{1\text{-}5} + \Delta G^\circ_{2\text{-}6}) - (\Delta G^\circ_{3\text{-}6} + \Delta G^\circ_{4\text{-}7}) + \Delta G^\circ_B - \Delta G^\circ_P$$

$$\Delta G^\circ_{rxn1} = \Delta G^\circ_{PT} - \Delta G^\circ_{BT}$$
$$\approx (\Delta G^\circ_{1\text{-}5} + \Delta G^\circ_{2\text{-}6}) - (\Delta G^\circ_{3\text{-}6} + \Delta G^\circ_{4\text{-}8}) + \Delta G^\circ_B - \Delta G^\circ_P$$

$$\Delta G^\circ_{rxn2} - \Delta G^\circ_{rxn1} = \Delta G^\circ_{4\text{-}8} - \Delta G^\circ_{4\text{-}7} = \Delta\Delta G^\circ$$

FIG. 2

FIG. 3

Forward Primer
CAGCCTCATCCAAAAGAGGAAA

$\Delta G°_{rxn1}$ = 3.38 kcal/mol

Blocker AAAAGAGGAAA C AGGACCCCAGAGCTC

Reverse Primer Sequence

. . . . . AGTGTCGGAGTAGGTTTTCTCCTTT A TCCTGGGGTCTCGAGGGA . . . . . . TTACGACCCCAAATCTCACTC . . . . .
Target NA18537

. . . . . AGTGTCGGAGTAGGTTTTCTCCTTT G TCCTGGGGTCTCGAGGGA . . . . . . TTACGACCCCAAATCTCACTC . . . . .
Variant NA18562

SMAD7 rs4939827

100% Target
Cq = 24.1

0.1% Target
Cq = 34.7

100% WT
Cq = 38.9
ΔCq = 14.8

RFU

Cycles

FIG. 4

| Extension Temperature (°C) | Target Cq | ΔCq |
|---|---|---|
| 56 | 28.4 | 12.5 |
| 58 | 24.7 | 8.9 |
| 60 | 25.3 | 13.2 |
| 62 | 26.7 | 13.5 |
| 64 | 36.8 | 17.0 |
| 66 | 58.7 | - |

Working Range: 56-64°C

Primer
TGTATATAGACGGTAAAATAAACACCAAGA

Blocker
ACACCAAGA<sup>C</sup>GTGGTAAATATTTACCTGGTC

. . . . . AACATATATCTGCCATTTTATTTGTGGTTCTCCACCATTTATAAATGGACCAGGGAC . . . . . .
Target                                                                NA18562

. . . . . AACATATATCTGCCATTTTATTTGTGGTTCTGCACCATTTATAAATGGACCAGGGAC . . . . . .
Variant                                                               NA18537

BRAF rs3789806

FIG. 5

FIG. 6A

| | Target Cq | ΔCq |
|---|---|---|
| Primer-Blocker Pair 1 | 25.3 | 13.2 |
| | 26.9 | 11.3 |
| Primer-Blocker Pair 2 | 24.3 | 14.0 |
| | 26.5 | 17.2 |

Primer-Blocker Pair 1
TGTATATAGACGGTAAAATAAACACCAAGA
ACACCAAGA<sup>C</sup>GTGGTAAATATTTACCTGGTC

Primer-Blocker Pair 2
GAGCCTTGTATATAGACGGTAAAATAAAC
CGGTAAAATAAACACCAAGA<sup>C</sup>GTGGTAAATATTTAC

Primer-Blocker Pair 3
TGGAGCCTTGTATATAGACGGTAAA
GACGGTAAAATAAACACCAAGA<sup>C</sup>GTGGTAAA

Primer-Blocker Pair 4
ACTGGAGCCTTGTATATAGACGG
GTATATAGACGGTAAAATAAACACCAAGA<sup>C</sup>GTGGT

......TTGACCTCGGAACATATATCTGCCATTTTATTTGTGGTTCT C CACCATTTATAAATGGACCAGGGAC......
Target

......TTGACCTCGGAACATATATCTGCCATTTTATTTGTGGTTCT G CACCATTTATAAATGGACCAGGGAC......
Variant

BRAF rs3789806

FIG. 6B

36

Simulation of Effects of $\Delta G°_{rxn1}$, Given Fixed $\Delta\Delta G° = 2$ kcal/mol

FIG. 7A

Experimental Validation on SMAD7 rs4939827

Primer
CAGCCTCATCCAAAAGAGGAAA

Blocker C1  AAAAGAGGAAA<sup>C</sup>AGGACCCCAGA

Blocker C2  AAAAGAGGAAA<sup>C</sup>AGGACCCCAGAG

Blocker C3  AAAAGAGGAAA<sup>C</sup>AGGACCCCAGAGC

Blocker C4  AAAAGAGGAAA<sup>C</sup>AGGACCCCAGAGCT

Blocker C5  AAAAGAGGAAA<sup>C</sup>AGGACCCCAGAGCTC

Blocker C6  AAAAGAGGAAA<sup>C</sup>AGGACCCCAGAGCTCC

| $\Delta G^{\circ}_{rxn1}$ (kcal/mol) | Target Cq | $\Delta$Cq |
|---|---|---|
| 0.13 | 23.2 | 6.1 |
| 0.73 | 23.3 | 10.9 |
| 2.19 | 23.6 | 12.9 |
| 2.78 | 23.7 | 15.4 |
| 3.38 | 24.8 | 17.4 |
| 4.54 | 25.6 | 14.7 |

......AGTGTCGGAGTAGGTTTTCTCCTTT|A|TCCTGGGGTCTCGAGGGA......
Target

......AGTGTCGGAGTAGGTTTTCTCCTTT|G|TCCTGGGGTCTCGAGGGA......
Variant

FIG. 7B(I)

Experimental Validation on SMAD7 rs4939827

Primer
CAGCCTCATCCAAAAGAGGAAA

Blocker T1
AAAAGAGGAAA<sup>T</sup>AGGACCCCAGA

Blocker T2
AAAAGAGGAAA<sup>T</sup>AGGACCCCAGAG

Blocker T3
AAAAGAGGAAA<sup>T</sup>AGGACCCCAGAGC

Blocker T4
AAAAGAGGAAA<sup>T</sup>AGGACCCCAGAGCT

Blocker T5
AAAAGAGGAAA<sup>T</sup>AGGACCCCAGAGCTC

Blocker T6
AAAAGAGGAAA<sup>T</sup>AGGACCCCAGAGCTCC

| $\Delta G^{\circ}_{rxn1}$ (kcal/mol) | Target Cq | $\Delta Cq$ |
|---|---|---|
| -2.16 | 23.3 | 1.7 |
| -1.57 | 23.5 | 6.6 |
| -0.11 | 25.8 | 10.1 |
| 0.49 | 27.3 | 9.3 |
| 1.08 | 29.9 | 8.8 |
| 2.24 | 32.6 | 8.8 |

.....AGTGTCGGAGTAGGTTTTCTCCTTT G TCCTGGGGTCTCGAGGGA......
Target

.....AGTGTCGGAGTAGGTTTTCTCCTTT A TCCTGGGGTCTCGAGGGA......
Variant

FIG. 7B(II)

Primer

TGTATATAGACGGTAAAATAAACACCAAGA

Blocker ACACCAAGACGTGGTAAATATTTACCTGGTC AAAA

. . . . . AACATATATCTGCCATTTTATTTGTGGTTCTCCACCATTTATAAATGGACCAGGGAC . . . . .
Target

. . . . . AACATATATCTGCCATTTTATTTGTGGTTCTGCACCATTTATAAATGGACCAGGGAC . . . . .
Variant

BRAF rs3789806

FIG. 8

FIG. 9A

FIG. 9B

Possible Nonspecific Binding of Primer and
Blocker to Other Templates in Solution

FIG. 10

Primer

CGATATGGTTTGTTTCAAGGTATGATTTTTA

Blocker-I

AAGGTATGATTTTTAATTAAAAATTGTTGTTCACATTTGAAGG

......TAGCTATACCAAACAAAGTTCCATACTAAAAATTAATTTTT `---` AACAAGTGTAAACTTCCA......

Target                                                            NA18562

......TAGCTATACCAAACAAAGTTCCATACTAAAAATTAATTTTT `AAC` AACAAGTGTAAACTTCCA......

Variant                                    Detect Deletion           NA18537

—NA18537
----NA18562

Target Cq = 26.3

$\Delta$Cq = 16.6

FIG. 11A

Primer

CGATATGGTTTGTTTCAAGGTATGATTTTTA

Blocker-D
AAGGTATGATTTTTAATTAAAAA - - - TTGTTCACATTTGAAGG

......TAGCTATACCAAACAAAGTTCCATACTAAAAATTAATTTTT AAC AACAAGTGTAAACTTCCA......

Target                                                                                    NA18537

......TAGCTATACCAAACAAAGTTCCATACTAAAAATTAATTTTT - - - AACAAGTGTAAACTTCCA......

Variant                                    Detect Insertion              NA18562

Target Cq = 27.8
ΔCq = 16.3

FIG. 11B

Primer GTGGTAAATATTTACCTGGTCCCTG

Blocker GGTAAAATAAACACCAAGA‑C‑GTGGTAAATATTTAC

......GCCATTTTATTTGTGGTTCTCCACCATTTATAAATGGACCAGGGACA......
Target

......GCCATTTTATTTGTGGTTCTGCACCATTTATAAATGGACCAGGGACA......
Variant          BRAF rs3789806

Target Cq = 23.9
ΔCq = 2.5

Target   Variant

**FIG. 12**

FIG. 13

Forward Primer
TGGAGCCTTGTATATAGACGGTAAAAT

Forward Blocker
GACGGTAAAATAAACACCAAGA̲C̲GTGGTAAA

...TGACCTCGGAACATATATCTGCCATTTTATTTGTGGTTCT[C]CACCATTTATAAATGGACCAGGGACAACAACTACAA... ⎤ Reverse
Target                                                                              ⎦ Template
...TGACCTCGGAACATATATCTGCCATTTTATTTGTGGTTCT[G]CACCATTTATAAATGGACCAGGGACAACAACTACAA...
Variant                                    BRAF rs3789806

...ACTGGAGCCTTGTATATAGACGGTAAAATAAACACCAAGA[C]GTGGTAAATATTTACCTGGTCCCTGTTGTTGATGTT... ⎤ Forward
Variant                                                                              ⎦ Template
...ACTGGAGCCTTGTATATAGACGGTAAAATAAACACCAAGA[G]GTGGTAAATATTTACCTGGTCCCTGTTGTTGATGTT...
Target
                                          TTGTGGTTCT̲G̲CACCATTTATAAATGGACCA
                                          Reverse Blocker
                                                                         TATAAATGGACCAGGGACAACAACTAC
                                                                         Reverse Primer

**FIG. 14**

**REFERENCES CITED IN THE DESCRIPTION**

**Patent documents cited in the description**

- US 62000114
- WO 26094700251 A
- US 2014017685 A
- WO 62000114 A

SEQUENCE LISTING

    <110> RICE UNIVERSITY
MARSHALL, WILLIAM

    <120> ALLELE-SPECIFIC AMPLIFICATION USING A COMPOSITION OF OVERLAPPING NON-ALLELE-SPECIFIC PRIMER AND ALLELE-SPECIFIC BLOCKER OLIGONUCLEOTIDES

    <130> 14-21013-WO (260947.00251)

    <140>
    <141>

    <150> 62/000,114
    <151> 2014-05-19

    <160> 49

    <170> PatentIn version 3.5

    <210> 1
    <211> 11
    <212> DNA
    <213> Artificial Sequence

    <220>
    <223> Description of Artificial Sequence: Synthetic primer

    <400> 1
agctgaccta a       11

    <210> 2
    <211> 11
    <212> DNA
    <213> Artificial Sequence

    <220>

<223> Description of Artificial Sequence: Synthetic oligonucleotide

<220>
<223> May be 3' C3 modified

<400> 2
acctaagcga t         11


<210> 3
<211> 22
<212> DNA
<213> Artificial Sequence

<220>
<223> Description of Artificial Sequence: Synthetic primer

<400> 3
cagcctcatc caaaagagga aa         22


<210> 4
<211> 27
<212> DNA
<213> Artificial Sequence

<220>
<223> Description of Artificial Sequence: Synthetic oligonucleotide

<220>
<223> May be 3' C3 modified

<400> 4
aaaagaggaa acaggacccc agagctc         27


<210> 5
<211> 30
<212> DNA
<213> Artificial Sequence

<220>
<223> Description of Artificial Sequence: Synthetic primer

<400> 5
tgtatataga cggtaaaata aacaccaaga         30


<210> 6
<211> 31
<212> DNA
<213> Artificial Sequence

<220>
<223> Description of Artificial Sequence: Synthetic oligonucleotide

<220>
<223> May be 3' C3 modified

<400> 6
acaccaagac gtggtaaata tttacctggt c         31

**EP 3 146 080 B1**

<210> 7
<211> 29
<212> DNA
<213> Artificial Sequence

<220>
<223> Description of Artificial Sequence: Synthetic primer

<400> 7
gagccttgta tatagacggt aaaataaac          29

<210> 8
<211> 36
<212> DNA
<213> Artificial Sequence

<220>
<223> Description of Artificial Sequence: Synthetic oligonucleotide

<220>
<223> May be 3' C3 modified

<400> 8
cggtaaaata aacaccaaga cgtggtaaat atttac          36

<210> 9
<211> 25
<212> DNA
<213> Artificial Sequence

<220>
<223> Description of Artificial Sequence: Synthetic primer

<400> 9
tggagccttg tatatagacg gtaaa          25

<210> 10
<211> 31
<212> DNA
<213> Artificial Sequence

<220>
<223> Description of Artificial Sequence: Synthetic oligonucleotide

<220>
<223> May be 3' C3 modified

<400> 10
gacggtaaaa taaacaccaa gacgtggtaa a          31

<210> 11
<211> 23
<212> DNA
<213> Artificial Sequence

<220>
<223> Description of Artificial Sequence: Synthetic primer

<400> 11
actggagcct tgtatataga cgg          23


<210> 12
<211> 35
<212> DNA
<213> Artificial Sequence


<220>
<223> Description of Artificial Sequence: Synthetic oligonucleotide


<220>
<223> May be 3' C3 modified


<400> 12
gtatatagac ggtaaaataa acaccaagac gtggt          35


<210> 13
<211> 23
<212> DNA
<213> Artificial Sequence


<220>
<223> Description of Artificial Sequence: Synthetic oligonucleotide


<220>
<223> May be 3' C3 modified


<400> 13
aaaagaggaa acaggacccc aga          23


<210> 14
<211> 24
<212> DNA
<213> Artificial Sequence


<220>
<223> Description of Artificial Sequence: Synthetic oligonucleotide


<220>
<223> May be 3' C3 modified


<400> 14
aaaagaggaa acaggacccc agag          24


<210> 15
<211> 25
<212> DNA
<213> Artificial Sequence


<220>
<223> Description of Artificial Sequence: Synthetic oligonucleotide


<220>
<223> May be 3' C3 modified


<400> 15
aaaagaggaa acaggacccc agagc          25

<210> 16
<211> 26
<212> DNA
<213> Artificial Sequence

<220>
<223> Description of Artificial Sequence: Synthetic oligonucleotide

<220>
<223> May be 3' C3 modified

<400> 16
aaaagaggaa acaggacccc agagct        26

<210> 17
<211> 28
<212> DNA
<213> Artificial Sequence

<220>
<223> Description of Artificial Sequence: Synthetic oligonucleotide

<220>
<223> May be 3' C3 modified

<400> 17
aaaagaggaa acaggacccc agagctcc        28

<210> 18
<211> 23
<212> DNA
<213> Artificial Sequence

<220>
<223> Description of Artificial Sequence: Synthetic oligonucleotide

<220>
<223> May be 3' C3 modified

<400> 18
aaaagaggaa ataggacccc aga        23

<210> 19
<211> 24
<212> DNA
<213> Artificial Sequence

<220>
<223> Description of Artificial Sequence: Synthetic oligonucleotide

<220>
<223> May be 3' C3 modified

<400> 19
aaaagaggaa ataggacccc agag        24

<210> 20
<211> 25

<212> DNA
<213> Artificial Sequence

<220>
<223> Description of Artificial Sequence: Synthetic oligonucleotide

<220>
<223> May be 3' C3 modified

<400> 20
aaaagaggaa ataggacccc agagc        25

<210> 21
<211> 26
<212> DNA
<213> Artificial Sequence

<220>
<223> Description of Artificial Sequence: Synthetic oligonucleotide

<220>
<223> May be 3' C3 modified

<400> 21
aaaagaggaa ataggacccc agagct        26

<210> 22
<211> 27
<212> DNA
<213> Artificial Sequence

<220>
<223> Description of Artificial Sequence: Synthetic oligonucleotide

<220>
<223> May be 3' C3 modified

<400> 22
aaaagaggaa ataggacccc agagctc        27

<210> 23
<211> 28
<212> DNA
<213> Artificial Sequence

<220>
<223> Description of Artificial Sequence: Synthetic oligonucleotide

<220>
<223> May be 3' C3 modified

<400> 23
aaaagaggaa ataggacccc agagctcc        28

<210> 24
<211> 35
<212> DNA
<213> Artificial Sequence

<220>
<223> Description of Artificial Sequence: Synthetic oligonucleotide

<400> 24
acaccaagac gtggtaaata tttacctggt caaaa        35

<210> 25
<211> 31
<212> DNA
<213> Artificial Sequence

<220>
<223> Description of Artificial Sequence: Synthetic primer

<400> 25
cgatatggtt tgtttcaagg tatgattttt a        31

<210> 26
<211> 43
<212> DNA
<213> Artificial Sequence

<220>
<223> Description of Artificial Sequence: Synthetic oligonucleotide

<220>
<223> May be 3' C3 modified

<400> 26
aaggtatgat ttttaattaa aaattgttgt tcacatttga agg        43

<210> 27
<211> 25
<212> DNA
<213> Artificial Sequence

<220>
<223> Description of Artificial Sequence: Synthetic primer

<400> 27
gtggtaaata tttacctggt ccctg        25

<210> 28
<211> 35
<212> DNA
<213> Artificial Sequence

<220>
<223> Description of Artificial Sequence: Synthetic oligonucleotide

<220>
<223> May be 3' C3 modified

<400> 28
ggtaaaataa acaccaagac gtggtaaata tttac        35

<210> 29
<211> 27

<212> DNA
<213> Artificial Sequence

<220>
<223> Description of Artificial Sequence: Synthetic primer

<400> 29
tggagccttg tatatagacg gtaaaat          27

<210> 30
<211> 31
<212> DNA
<213> Artificial Sequence

<220>
<223> Description of Artificial Sequence: Synthetic oligonucleotide

<220>
<223> May be 3' C3 modified

<400> 30
accaggtaaa tatttaccac gtcttggtgt t          31

<210> 31
<211> 27
<212> DNA
<213> Artificial Sequence

<220>
<223> Description of Artificial Sequence: Synthetic primer

<400> 31
catcaacaac agggaccagg taaatat          27

<210> 32
<211> 21
<212> DNA
<213> Artificial Sequence

<220>
<223> Description of Artificial Sequence: Synthetic primer

<400> 32
ctcactctaa accccagcat t          21

<210> 33
<211> 16
<212> DNA
<213> Artificial Sequence

<220>
<223> Description of Artificial Sequence: Synthetic oligonucleotide

<400> 33
atcgcttagg tcagct          16

<210> 34
<211> 16

<212> DNA
<213> Artificial Sequence

<220>
<223> Description of Artificial Sequence: Synthetic oligonucleotide

<400> 34
atcacttagg tcagct        16

<210> 35
<211> 44
<212> DNA
<213> Artificial Sequence

<220>
<223> Description of Artificial Sequence: Synthetic oligonucleotide

<400> 35
agggagctct ggggtcctat ttcctctttt ggatgaggct gtga        44

<210> 36
<211> 44
<212> DNA
<213> Artificial Sequence

<220>
<223> Description of Artificial Sequence: Synthetic oligonucleotide

<400> 36
agggagctct ggggtcctgt ttcctctttt ggatgaggct gtga        44

<210> 37
<211> 57
<212> DNA
<213> Artificial Sequence

<220>
<223> Description of Artificial Sequence: Synthetic oligonucleotide

<400> 37
cagggaccag gtaaatattt accacctctt ggtgtttatt ttaccgtcta tatacaa        57

<210> 38
<211> 57
<212> DNA
<213> Artificial Sequence

<220>
<223> Description of Artificial Sequence: Synthetic oligonucleotide

<400> 38
cagggaccag gtaaatattt accacgtctt ggtgtttatt ttaccgtcta tatacaa        57

<210> 39
<211> 67
<212> DNA
<213> Artificial Sequence

<220>
<223> Description of Artificial Sequence: Synthetic oligonucleotide

<400> 39

```
cagggaccag gtaaatattt accacctctt ggtgtttatt ttaccgtcta tatacaaggc       60

tccagtt                                                                 67
```

<210> 40
<211> 67
<212> DNA
<213> Artificial Sequence

<220>
<223> Description of Artificial Sequence: Synthetic oligonucleotide

<400> 40

```
cagggaccag gtaaatattt accacgtctt ggtgtttatt ttaccgtcta tatacaaggc       60

tccagtt                                                                 67
```

<210> 41
<211> 59
<212> DNA
<213> Artificial Sequence

<220>
<223> Description of Artificial Sequence: Synthetic oligonucleotide

<400> 41
accttcaaat gtgaacaatt tttaattaaa aatcatacct tgaaacaaac catatcgat       59

<210> 42
<211> 62
<212> DNA
<213> Artificial Sequence

<220>
<223> Description of Artificial Sequence: Synthetic oligonucleotide

<400> 42

```
accttcaaat gtgaacaaca atttttaatt aaaaatcata ccttgaaaca aaccatatcg       60

at                                                                      62
```

<210> 43
<211> 40
<212> DNA
<213> Artificial Sequence

<220>

<223> Description of Artificial Sequence: Synthetic oligonucleotide

<220>
<223> May be 3' C3 modified

<400> 43
aaggtatgat ttttaattaa aaattgttca catttgaagg        40

<210> 44
<211> 47
<212> DNA
<213> Artificial Sequence

<220>
<223> Description of Artificial Sequence: Synthetic oligonucleotide

<400> 44
acagggacca ggtaaatatt taccacctct tggtgtttat tttaccg        47

<210> 45
<211> 47
<212> DNA
<213> Artificial Sequence

<220>
<223> Description of Artificial Sequence: Synthetic oligonucleotide

<400> 45
acagggacca ggtaaatatt taccacgtct tggtgtttat tttaccg        47

<210> 46
<211> 77
<212> DNA
<213> Artificial Sequence

<220>
<223> Description of Artificial Sequence: Synthetic oligonucleotide

<400> 46

```
aacatcaaca acagggacca ggtaaatatt taccacctct tggtgtttat tttaccgtct        60

atatacaagg ctccagt        77
```

<210> 47
<211> 77
<212> DNA
<213> Artificial Sequence

<220>
<223> Description of Artificial Sequence: Synthetic oligonucleotide

<400> 47

```
aacatcaaca acagggacca ggtaaatatt taccacgtct tggtgtttat tttaccgtct      60

atatacaagg ctccagt                                                      77
```

```
<210> 48
<211> 77
<212> DNA
<213> Artificial Sequence

<220>
<223> Description of Artificial Sequence: Synthetic oligonucleotide

<400> 48
```

```
actggagcct tgtatataga cggtaaaata aacaccaaga cgtggtaaat atttacctgg      60

tccctgttgt tgatgtt                                                      77
```

```
<210> 49
<211> 77
<212> DNA
<213> Artificial Sequence

<220>
<223> Description of Artificial Sequence: Synthetic oligonucleotide

<400> 49
```

```
actggagcct tgtatataga cggtaaaata aacaccaaga ggtggtaaat atttacctgg      60

tccctgttgt tgatgtt                                                      77
```